(19) Europäisches Patentamt
European Patent Office
Office européen des brevets

(11) EP 4 424 658 A1

(12) EUROPEAN PATENT APPLICATION

(43) Date of publication:
**04.09.2024 Bulletin 2024/36**

(21) Application number: **23159632.1**

(22) Date of filing: **02.03.2023**

(51) International Patent Classification (IPC):
***C07C 1/32*** *(2006.01)* ***C07C 9/04*** *(2006.01)*
***C07C 9/06*** *(2006.01)* ***C07C 9/14*** *(2006.01)*
***C07C 9/08*** *(2006.01)* ***C07C 9/10*** *(2006.01)*
***C10G 1/00*** *(2006.01)*

(52) Cooperative Patent Classification (CPC):
(C-Sets available)
**C07C 1/323; C10G 1/086; C10G 1/10;**
C07C 2523/10; C07C 2523/42; C07C 2523/46

(Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **ECOLE POLYTECHNIQUE FEDERALE DE LAUSANNE (EPFL)
1015 Lausanne (CH)**

(72) Inventors:
- **WU, XinBang
1024 ECUBLENS VD (CH)**
- **DYSON, Paul
1024 ECUBLENS VD (CH)**
- **LEE, Wei-Tse
1024 ECUBLENS VD (CH)**

(74) Representative: **Cohausz & Florack Patent- & Rechtsanwälte Partnerschaftsgesellschaft mbB
Bleichstraße 14
40211 Düsseldorf (DE)**

# (54) METHOD FOR CONVERTING NITROGEN-CONTAINING POLYMERS INTO HYDROCARBON COMPOUNDS

(57) The invention relates to a method for converting a polymer into hydrocarbon compounds, comprising the steps of a. contacting the polymer with a catalyst to provide a reaction mixture, wherein the polymer contains nitrogen in the polymer backbone, wherein the catalyst comprises a transition metal on a carrier, b. heating the reaction mixture in the presence of a reducing agent to a temperature of 50 to 1000°C, c. recovering the hydrocarbon compounds from the reaction mixture.

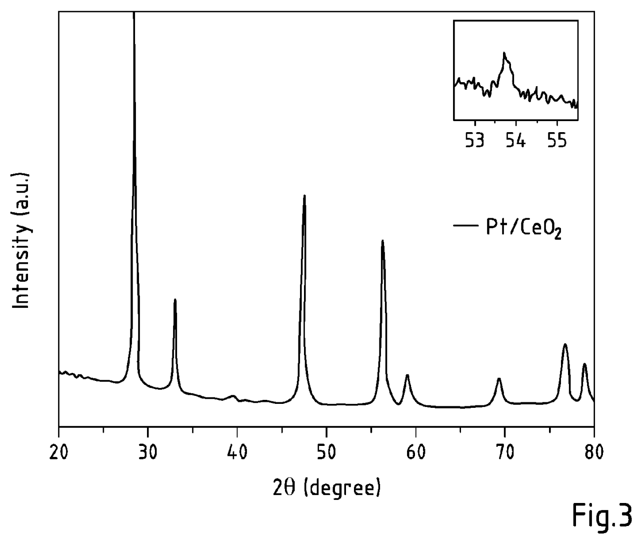


Fig.3

EP 4 424 658 A1

(52) Cooperative Patent Classification (CPC): (Cont.)

C-Sets
**C07C 1/323, C07C 9/04;**
**C07C 1/323, C07C 9/06;**
**C07C 1/323, C07C 9/08;**
**C07C 1/323, C07C 9/10;**
**C07C 1/323, C07C 9/14**

## Description

The present invention relates to a method for the direct conversion of polymers into hydrocarbon compounds.

The improper disposal of polymer waste leads to serious environmental issues due to its persistence in the environment. Polymers containing carbon-nitrogen linkages, such as polyamides (PAs), have a particularly high mechanical durability, thanks to stabilization through extensive inter-chain hydrogen bonding. This durability allows PAs to have diverse applications in the automotive, textile, fishing and engineering industries. However, this same durability also confers non-biodegradability, resulting in a long lifetime during which pollutants are leached into the environment. The effects of PA pollution are further exacerbated by current waste management systems, which offer only temporary solutions by disposal in landfill. Other methods to process waste PAs, such as through incineration or mechanical recycling, encounter significant challenges: incineration produces hazardous compounds, such as polycyclic aromatic hydrocarbons or microplastics, whereas mechanical recycling weakens the polymer after each cycle, decreasing the quality of the plastic and limiting its applicability.

Catalytic hydrogenolysis has the potential to convert mixed plastic waste to valuable products. Catalytic hydrogenolysis has been widely reported for the conversion of polyethylene (PE) and polypropylene (PP) to shorter chain alkanes (see for example Celik, G.; Kennedy, R. M.; Hackler, R. A.; Ferrandon, M.; Tennakoon, A.; Patnaik, S.; LaPointe, A. M.; Ammal, S. C.; Heyden, A.; Perras, F. A.; Pruski, M.; Scott, S. L.; Poeppelmeier, K. R.; Sadow, A. D.; Delferro, M. ACS Cent. Sci. 2019, 5 (11), 1795-1803; Chen, L.; Zhu, Y.; Meyer, L. C.; Hale, L. V.; Le, T. T.; Karkamkar, A.; Lercher, J. A.; Gutiérrez, O. Y.; Szanyi, J. React. Chem. Eng. 2022, 7 (4), 844-854; Kots, P. A.; Liu, S.; Vance, B. C.; Wang, C.; Sheehan, J. D.; Vlachos, D. G. ACS Catal. 2021, 11 (13), 8104-8115). The resulting products typically comprise a distribution of hydrocarbons in different phases, including gaseous C1-C4 hydrocarbons, liquid C5-C16 hydrocarbons, and solid C17-C34 hydrocarbons, depending on the conditions.

It should be noted that hydrogenolysis of polyolefins only involves cleaving C-C bonds and does not account for the selective cleavage of carbon-heteroatom bonds. On the other hand, selectivity is especially important for the conversion of heteroatom containing substrates, as they can form a multitude of by-products. Since polymers containing nitrogen in the polymer backbone such as PAs are frequently used due to their durability that is a problem when it comes to the recycling of the PAs, a method for the recycling of polymers containing nitrogen in the polymer backbone would be desirable. In particular, it would be desirable to develop a method that allows to obtain products by hydrogenolysis that are as uniform as possible. More particularly, it would be more desirable if the hydrocarbon products are predominantly liquid.

Against this background, it is an object of the present invention to provide a method for the conversion of polymers containing nitrogen in their backbone to products that are as uniform as possible. The method should preferably cleave C-C bonds only to a small extent but cleave C-N bonds. The method should preferably further be robust and/or applicable on a large scale.

The object described above is solved in accordance with the present invention by a method for converting a polymer into hydrocarbon compounds, comprising the steps of

a. contacting the polymer with a catalyst to provide a reaction mixture,

    wherein the polymer contains nitrogen in the polymer backbone,
    wherein the catalyst comprises a transition metal on a carrier,

b. heating the reaction mixture in the presence of a reducing agent to a temperature of 50 to 1000°C,
c. recovering the hydrocarbon compounds from the reaction mixture.

Thus, it was surprisingly found that by heating a transition metal on a carrier and a polymer containing nitrogen in the polymer backbone in the presence of reducing agent to 150 to 1000°C, the polymer was converted to hydrocarbon compounds. It was particularly surprising that only little cleavage of C-C bonds was observed.

In the method according to the invention, a polymer that contains nitrogen in the polymer backbone is used as the starting material. The polymer backbone is the main chain of a polymer. For example in polypropylene, the polymer backbone is $-(CH_2)-$ and the side chains are $-CH_3$. A polymer backbone can also contain heteroatoms. For example in polyethers, the polymer backbone contains oxygen atoms. In the polymers used in the invention, the polymer backbone contains nitrogen atoms.

Various embodiments of the method according to the invention are described in the following. The individual embodiments may be combined with each other at will.

The method according to the invention can be applied to different polymers containing nitrogen in the polymer backbone. The polymer containing nitrogen in the polymer backbone may also be present in a mixture with polymers that do not contain nitrogen. The repeat units of the polymer containing nitrogen in the polymer backbone may contain

saturated aliphatic hydrocarbon moieties, unsaturated aliphatic hydrocarbon moieties and/or aromatic hydrocarbon moieties, each of which may be substituted, for example with $C_1$-$C_4$-alkyl groups, halogens, in particular -Cl, - Br and -I, =O, =S, =NH, or -CN. Examples for saturated aliphatic hydrocarbon moieties are $-(CH_2)-$ and $-(CH_2CH(CH_3))-$. Examples for unsaturated aliphatic hydrocarbon moieties are $-(C_3H_4)-$ and $-(C_4H_6)-$. Examples for aromatic moieties are $-(C_6H_4)-$ and $-(C_{10}H_6)-$. Preferably, the polymer comprises N-C=O moieties.

The polymer containing nitrogen in the polymer backbone may also contain further heteroatoms, such as oxygen and/or sulfur, in the polymer backbone.

The polymer may also contain further additives such as glass, metal and carbon, for example glass fibers, metal fibers and carbon fibers. Thus, the polymer may also be a composite containing glass, metal and/or carbon.

In an embodiment of the invention the polymer is selected from the group consisting of polyamides, polyurethanes, polyureas, polyamines, aminoplast resins, and copolymers and mixtures thereof. Preferably the polymer is selected from the group consisting of polyamides, polyurethanes, and copolymers and mixtures thereof. According to a preferred embodiment, the polymer is a polyamide.

Examples for polyamides are polyamide-6 or poly(e-caprolactam) (PA6), polyamide-6,6 or poly(hexamethylene adipamide) (PA6,6), poly(11-aminoundecanoamide) (PA11), poly(dodecanolactam) (PA12), poly(tetramethylene adipamide) (PA4,6), poly(pentamethylene sebacamide) (PA5,10), poly(hexamethylene nonanediamide) (PA6,9), poly(hexamethylene sebacamide) (PA6,10), poly(hexamethylene dodecanoamide) (PA6,12), poly(m-xylylene adipamide) (PAMXD6), polyhexamethylene adipamide/polyhexamethyleneterephtalamide copolymer (PA66/6T), and polyhexamethylene adipamide/ polyhexamethyleneisophtalamide copolymer (PA66/6I). As explained above, this includes glass, metal and carbon-based polyamide composites.

Polyurethanes can be obtained by reaction of a diisocyanate with a diol. As diisocyanate, hexamethylene diisocyanate, toluene diisocyanate, methylene diphenyl diisocyanate can be used. The diol can be a polyether diol or a polyester diol.

Polyureas can be obtained by reaction of a diisocyanate with a diamine. As diisocyanate, hexamethylene diisocyanate, toluene diisocyanate, methylene diphenyl diisocyanate can be used. The diamine can be aromatic or aliphatic.

Examples for polyamines are $-(HN(CH_2)_x)_m-$ with x being an integer from 1 to 10, such as $-(HNCH_2CH_2CH_2CH_2)_m-$ or $-(HNCH_2CH_2CH_2CH_2CH_2CH_2)_m-$, and - $(HN(CH_2(CH_3))_y)_m-$ with y being an integer from 1 to 10.

Aminoplast resins can be obtained by polycondensation of carbonyl compounds such as formaldehyde with compounds having NH groups, such as urea, melamine, or 2-cyanoguanidine.

The polymer containing nitrogen in the polymer backbone can be found in textile fibers, plastic tubes, plastic films, plastic sheets, fishing nets and lines, or plastic packaging such as flasks, bottles, blister packs, etc. In some embodiments, the polymer is not pretreated (no pretreatment is required, the polymer as such is put in contact with the catalyst). In other embodiments, the polymer can undergo a pretreatment, such as cutting, crushing, chemical pretreatment, etc.

The method according to the invention employs a transition metal. In an embodiment of the invention, the transition metal is selected from iron group metals, cobalt group metals, nickel group metals, and mixtures thereof. Preferably, the transition metal is selected from the group consisting of iron, cobalt, nickel, ruthenium, rhodium, platinum, iridium, and mixtures thereof. More preferably, the transition metal is platinum or iridium. Most preferably, the transition metal is platinum. It was found that when using platinum, a good conversion was obtained. Further, with platinum as a catalyst, mainly the carbon-heteroatom bonds, in particular carbon-nitrogen bonds, were cleaved.

The transition metal can be present in different forms. In an embodiment of the invention, the transition metal is present in the form of a transition metal cluster. In another embodiment of the invention, the transition metal is present as a transition metal oxide. The transition metal may be present as a nanoparticle with a size of 5 to 15 nm.

Different carriers for the transition metal can be used in the present invention. In an embodiment of the invention, the carrier is zirconia, ceria, alumina, silica, zeolites such as zeolite-Y or ZSM-5, or carbon, preferably ceria or carbon. Most preferably, the carrier is ceria.

In the method according to the invention, the reaction mixture is heated to 50 to 1000°C in the presence of a reducing agent. In an embodiment of the invention, the reducing agent is selected from the group consisting of hydrogen, sodium hydride, hydrazine, formic acid, hydrogen donors such as alcohols, for example isopropanol or glycerol, and saturated alkanes such as polyethylene, and mixtures thereof. The use of polyethylene as reducing agent is for example described in F. Zhang et al. Science, 2020, 370 (6515), 437-441. Preferably, the reducing agent is selected from the group consisting of hydrogen, sodium hydride, hydrazine, formic acid, isopropanol, glycerol, and mixtures thereof. More preferably, the reducing agent is hydrogen. According to a preferred embodiment, the reducing agent is hydrogen at a pressure from 1 to 200 bars, preferably from 25 to 150 bars, more preferably from 40 to 100 bars, most preferably from 40 to 60 bars. It was found that at the aforementioned pressures, a good conversion was obtained, while the method could be conducted safely.

In the method according to the invention, the reaction mixture is heated in step b. to a temperature of 50 to 1000°C. In an embodiment of the invention, the reaction mixture is heated in step b. to a temperature of 150 to 500°C, preferably from 200 to 500°C, more preferably from 250 to 400°C, even more preferably from 300 to 400°C, most preferably from 300 to 375 °C. It was found that at these temperatures, the reaction proceeded with good conversion and good selectivity of carbon-carbon versus carbon-heteroatom cleavage, in particular carbon-nitrogen cleavage.

The method according to the invention can be conducted without a solvent or in the presence of a solvent. In an embodiment of the invention, step a. is conducted without a solvent. A solvent recovery and disposal are, thus, not necessary.

Further, also step b. may be conducted without a solvent.

According to an embodiment, step a. and step b. are conducted without a solvent. In some embodiments, the products, i.e. the hydrocarbon compounds, act as the solvent.

In an embodiment of the invention, step a. is conducted in a solvent. Different solvents may be used in this embodiment. Good results have been obtained when step a. is conducted in an organic solvent. The organic solvent may in particular be C5-C16 saturated aliphatic compounds, preferably C8-C12 saturated aliphatic compounds, or alcohols, in particular methanol, ethanol, propanol, isopropanol. Examples of C6-C16, in particular C8-C12, saturated aliphatic compounds are octane, none, decane, undecane, and dodecane.

In the method according to the invention, the polymer containing nitrogen compounds in the polymer backbone is converted to hydrocarbon compounds that are recovered from the reaction mixture. The hydrocarbon compounds can be different types of hydrocarbon compounds, such as aliphatic compounds, in particular alkane, alkene, cycloalkane, cycloalkene, or aromatic compounds. The hydrocarbon compounds can be of different lengths. In an embodiment of the invention, the hydrocarbon compounds have 1 to 20 carbon atoms, preferably 2 to 20 carbon atoms. The hydrocarbon compounds are preferably selected from the group consisting of alkanes, cycloalkanes, alkenes, aromatic hydrocarbon compounds, and mixtures thereof. More preferably, the hydrocarbon compounds are selected from the group consisting of alkanes, cycloalkanes, aromatic hydrocarbon compounds, and mixtures thereof. Most preferably, the hydrocarbon compounds are selected from the group consisting of alkanes, cycloalkanes and mixtures thereof.

In an embodiment of the invention, the alkanes, in particular the alkanes of the hydrocarbon compounds, may have 1 to 20 carbon atoms, preferably 2 to 20 carbon atoms, more preferably 4 to 20 carbon atoms.

In another embodiment of the invention, the cycloalkanes in particular the cycloalkanes of the hydrocarbon compounds, have 3 to 20 carbon atoms, preferably 3 to 10 carbon atoms.

In another embodiment of the invention, the alkenes have 2 to 20 carbon atoms, preferably 4 to 20 carbon atoms.

In yet another embodiment of the invention, the aromatic hydrocarbon compounds have 6 to 20 carbon atoms, preferably 6 to 15 carbon atoms.

The method according to the invention has the advantage that it allows to reduce the carbon-carbon bond cleavage, in particular when using catalysts such as iridium or platninum, thus yielding a lower amount of methane and a larger amount of longer hydrocarbon compounds. In an embodiment of the invention, the hydrocarbon compounds comprise methane in an amount of 60 wt.% or less, preferably 40 wt.% or less, more preferably 20 wt.% or less, most preferably 5 wt.% or less, based on the total weight of the hydrocarbon compounds.

In this way, the method according to the invention yields lower amounts of methane and larger amounts of longer hydrocarbon compounds, in particular liquid hydrocarbon compounds such as C5 to C12 hydrocarbon compounds.

The size of the hydrocarbon compounds obtained is limited by the size of the continuous carbon chains in the repeat units of the polymer containing nitrogen. For example, in polyamide-6, the largest continuous carbon chain contains six carbon atoms, hence the hydrocarbon compounds may contain hexane. In poly(dodecanolactam), the largest continuous carbon chain contains twelve carbon atoms, hence the hydrocarbon compounds may contain dodecane.

In an embodiment of the invention, the polymer comprises at least one type of repeat units and the hydrocarbon compounds comprise an alkane, a cycloalkane, or an aromatic compound having the same number of carbon atoms as the at least one type of repeat units of the polymer, in an amount of at least 10 wt.%, preferably in an amount of at least 20 wt.%, more preferably in an amount of at least 30 wt.%, even more preferably in an amount of at least 40 wt.%, most preferably in an amount of at least 50 wt.%, based on the total weight of the hydrocarbon compound.

The method according to the invention may be conducted in continuous, semi-continuous, or batch-wise fashion. In particular, the method according to the invention may be conducted under semi-continuous flow or continuous flow conditions. Flow reactors may allow the hydrocarbon products to be distilled and separated based on their molecular weight (i.e. C5-C7, C8-C10, C11-C13, and so on). Furthermore, flow reactors may allow to minimize C-C bond cleaving such that hydrocarbon compounds with more carbon atoms, in particular C6 or C12, may be obtained, depending on the hydrocarbon chain length of the repeat unit in the nitrogen-containing polymer.

The method according to the invention, in particular step b., may be conducted for different periods of time. In an embodiment of the invention, step b. is conducted for 2 to 80 hours, preferably 5 to 60 hours, more preferably 8 to 48 hours, most preferably 10 to 36 hours. This is particularly the case when conducting the reaction in batch-wise.

It was found that when conducting step b. for a period of time as mentioned above, the method could be conducted with good conversion in a reasonable time frame.

The transition metal used in the method according to the invention can be present in various amounts. In an embodiment of the invention, the transition metal is present in an amount of 0.001 to 5 wt.%, preferably 0.01 to 5 wt.%, more preferably 0.05 to 3 wt.%, most preferably 0.1 to 2 wt.%, based on the amount of the polymer.

When conducting the reaction with the aforementioned amounts of the transition metal, the reaction proceeded sufficiently fast, while it also was possible to limit the amount of catalyst employed.

In the method according to the invention, different bonds are preferably cleaved for converting the polymer into hydrocarbon compounds. Advantageously, carbon-nitrogen bonds are cleaved. Carbon-oxygen bonds may also be cleaved. In some cases, carbon-carbon bonds may need to be cleaved. The cleaving of the bonds preferably takes place in the presence of the reducing agent. In particular, cleavage of one bond requires one equivalent of the reducing agent. The amount of the reducing agent, therefore, may be determined based on the number of bonds that need at least to be cleaved for converting the polymer into hydrocarbon compounds. For example, for a polymer containing nitrogen in the backbone and no further heteroatoms, at least the two bonds to the nitrogen atom in the backbone need to be cleaved for converting the polymer into hydrocarbon compounds, hence at least two equivalents of the reducing agent are needed, based on the bonds that need to be cleaved for the conversion. For converting a polyamide, at least the bonds to the nitrogen in the backbone and the bond to the oxygen atom need to be cleaved per repeat unit. Hence for a polyamide, at least three equivalents of the reducing agent are needed, based on the bonds that need to be cleaved for the conversion. The type and length of the hydrocarbon compounds formed is in particular affected by the bonds that are cleaved. For example, when carbon-carbon bonds are cleaved, shorter hydrocarbon compounds are obtained.

The reducing agent may be used in substoichiometric amounts or in excess, based on the number of carbon-nitrogen, carbon-oxygen and carbon-carbon bonds that need to be cleaved. According to a preferred embodiment, the reducing agent is used in substoichoimetric amounts, based on the number of carbon-nitrogen, carbon-oxygen and carbon-carbon bonds that need to be cleaved. In this way, cleaving large amounts of carbon-carbon bonds can be avoided and longer chain-liquid products can be obtained. Preferably, the reducing agent is present in amount of from 2 to 100 equivalents, more preferably from 2 to 20 equivalents, based on the amount of carbon-nitrogen, carbon-oxygen and carbon-carbon bonds that need to be cleaved. If the reducing agent is used in substoichiometric amounts, the transition metal may be any of the aforementioned transition metals, preferably platinum, iridium and/or ruthenium.

According to an embodiment of the invention, the transition metal is selected from rhodium, platinum and/or iridium, in particular platinum, and in step b., the reducing agent is hydrogen at a pressure of 25 to 200 bars. The reaction mixture is advantageously heated to 150 to 500°C, preferably from 200 to 500°C, more preferably from 250 to 400°C, even more preferably from 300 to 400°C, most preferably from 300 to 375 °C.

According to another embodiment of the invention, the transition metal is selected from ruthenium, rhodium, platinum and/or iridium, in particular ruthenium, and in step b., the reducing agent is hydrogen at a pressure of 1 to 50 bars, preferably 1 to 20 bars, more preferably 5 to 15 bars. The reaction mixture is advantageously heated to 150 to 500°C, preferably from 150 to 400°C, more preferably from 150 to 300°C, even more preferably from 150 to 250°C, most preferably from 200 to 250 °C.

Before recovering the hydrocarbon compounds from the reaction mixture, the reaction mixture is preferably cooled. In an embodiment of the invention, the reaction mixture is cooled to 0 to 50°C, preferably 10 to 30°C in an additional step after step b. and before step c.

In addition to the hydrocarbon compounds, other products of the conversion may be recovered. In an embodiment of the invention, ammonia and/or hydrocarbon amines, in particular ammonia, is recovered as additional product.

In some embodiments of the method according to the invention, the polymer and the catalyst are introduced into a reaction vessel, such as a reactor, simultaneously or subsequently. In further embodiments of the method according to the invention, the polymer and the catalyst are introduced into the reaction vessel, such as a reactor, batch-wise or continuously. In other embodiments of the method according to the invention, the polymer is continuously introduced into the reaction vessel, such as a reactor, that already contains the catalyst. In a preferred embodiment of the method according to the invention, the method is conducted continuously, for example by continuously flow. In continuous flow, the hydrocarbon products can be constantly removed, thus avoiding forming short-chain products.

The invention further relates to a method for preparing a catalyst comprising a transition metal, in particular a transition metal selected from the group consisting of rhodium, platinum, iridium, and mixtures thereof, on a carrier, comprising the steps of

a) Providing a carrier,
b) Preparing a mixture of a transition metal salt and the carrier in a solvent,
c) Applying thermal and/or mechanical energy to the mixture,
d) Removing the solvent to obtain a precatalyst powder, and
e) Calcining the precatalyst powder to obtain the catalyst.

Preferably, the carrier is defined as the carrier described herein.

Preferably, the transition metal is defined as the transition metal described herein.

Preferably, transition metal salt is a transition metal acetyl acetonate or a transition metal halide, in particular a chloride, wherein the transition metal is preferably selected from rhodium, platinum and/or iridium, more preferably platinum.

Preferably, the solvent is an organic solvent or water, preferably water.

Preferably, the thermal and/or mechanical energy is applied in the form of stirring or ultrasound, preferably ultrasound.

Preferably, the calcination in step e) is performed at a temperature of 400°C to 700°C, more preferably from 500°C to 600°C. The calcination in step e) is preferably conducted for 1 h to 15 h, more preferably 2 h to 10 h, most preferably from 3 h to 7 h. The calcination is preferably performed in air.

According to an embodiment, step e) is conducted at a temperature above 550°C.

According to an embodiment of the method for preparing a catalyst, a pre-annealing step is conducted before the calcination step e). The pre-annealing step may be conducted at a temperature of 200°C to 400°C, more preferably from 250°C to 350°C. The pre-annealing step may be conducted for 1 h to 15 h, more preferably 2 h to 10 h, most preferably from 3 h to 7 h. The pre-annealing step is preferably performed in an inert gas atmosphere, more preferably under a flow of argon. After the pre-annealing step, a washing step may be conducted, preferably using the same solvent as for the reaction.

According to another embodiment of the method for preparing a catalyst, a washing step is conducted before the calcination step e). The washing step may be conducted after the pre-calcination step. Water or the same solvent as used for the reaction may be used in the washing step.

Thus, according to an embodiment, the method contains the following additional steps after step d) and before step e):

d1) pre-annealing the precatalyst powder at a temperature of 300°C or less, preferably from 250°C to 300°C, and
d2) washing the product from step d1) with the solvent.

The washed product from step d2) is then calcined in step e) to obtain the catalyst.

Further features and advantages of the method emerge from the Examples described below that do not limit the invention and are intended to further illustrate the invention.

EXAMPLES

MATERIALS

All chemicals were used as purchased without further purification.

$RuCl_3 \cdot 3H_2O$ and $K_2PtCl_4$, $PdCl_2$, $IrCl_3 \cdot 3H_2O$ and $RhCl_3 \cdot 3H_2O$ were purchased from PreciousMetalsOnline. CeOz and 5% Ru/C were purchased from Sigma-Aldrich. 5% Pt/C was purchased from Alfa Aesar. p-Xylene was purchased from Acros Organics. Polyethylene (resins, Mn = g/mol), PA-6 (resins, Mn = 27,380 g/mol), PA-66 (resins, Mn = 47,240 g/mol), PA-12 (resins, Mn = 27,060 g/mol), PA-612 (resins, Mn = 14,730 g/mol), 30% carbon-fibre PA-66 composite (resins, Mn = 19,920 g/mol) and 30% glass-fibre reinforced PA-6 rods were purchased from Sigma-Aldrich. The rods were sawn into smaller pieces before reaction. Ultramid® C40 L (resins, Mn = 49,010 g/mol) was provided by BASF.

Synthesis of the catalyst M/CeOz

A modified two-step annealing procedure (based on Hai, X.; Xi, S.; Mitchell, S.; Harrath, K.; Xu, H.; Akl, D. F.; Kong, D.; Li, J.; Li, Z.; Sun, T.; Yang, H.; Cui, Y.; Su, C.; Zhao, X.; Li, J.; Pérez-Ramírez, J.; Lu, J. Nature Nanotechnology 2021, https://doi.org/10.1038/s41565-021-01022-y) was used to obtain ultra-high-density atomic clusters deposited on CeOz.

$K_2PtCl_4$ (0.213 g) and CeOz nanopowder (1 g, < 25 nm, BET) were dispersed in ultrapure water (20 mL) and sonicated for 10 min, and the water was then removed by rotary evaporator. The resulting powder was calcined at 300 °C in a tube furnace at a heating rate of 5 °C/min, for 5 h under a flow of argon (200 mL/min). After cooling to room temperature, the powder was washed with ultrapure water (2 x 15 mL), then filtered and dried under vacuum. The powder was then calcined in a tube furnace at 550 °C, at a heating rate of 1 °C/min, then for 5 h under static air to produce Pt/CeOz.

The same procedure using $RuCl_3 \cdot 3H_2O$ (0.259 g), $RhCl_3 \cdot 3H_2O$ (0.256 g), PdClz (0.167 g) and $IrCl_3.3H_2O$ (0.184 g) was performed for synthesis of the Ru/CeOz, Rh/CeOz, Pd/CeOz and Ir/CeOz, respectively.

Characterization of the catalyst M/CeOz (M = Rh, Ir, Pt, Ru, Pd)

Powder X-ray diffraction (PXRD) of all M/CeOz were measured from 2θ = 20° to 80° on a Bruker D8 Discover Vario diffractometer by using Cu Kα (L = 1.54 Å) radiation that was operated at 40 kV and 40 mA. X-Ray Photoelectron Spectroscopy (XPS) measurements were carried out on an Axis Supra (Kratos Analytical) using the monochromated Kα X-ray line of an Aluminium anode. The pass energy was set to 40 eV with a step size of 0.15 eV. The samples were electrically insulated from the sample holder and an electron flood gun was used to neutralize the charges. The binding energy scale was then referenced at 284.8 eV using the aliphatic line of the C1s orbital. Inductively coupled plasma mass spectrometry (ICP-MS) was performed on a Perkin Elmer NexIon 350 spectrometer for elemental analysis. Scanning transmission electron microscopy (STEM) images for Rh/CeOz and Ir/CeOz were captured on a FEI Tecnai Osiris electron microscope at 200 KeV, while high resolution (HR) STEM images for Pt/CeOz were captured on a FEI Titan Themis electron microscope at 300 keV. High-angle annular dark field (HAADF) imaging mode was used for all STEM imaging. Energy-dispersive X-ray spectroscopy (EDX) was performed at 20 KeV

The PXRD diffractogram for the catalyst comprising rhodium on ceria (Rh/CeOz, see Fig. 1) only showed the peaks for CeOz.

The PXRD diffractogram for the catalyst comprising iridium on ceria (Ir/CeOz, see Fig. 2) showed IrOz peaks at 2θ = 34.5° (101), 39.7° (200), 53.7° (211) and 65.6 (112) in addition to the peaks for CeOz. The IrOz peaks are shown in the insets of Fig. 2.

The PXRD diffractogram for the catalyst comprising platinum on ceria (Pt/CeOz, see Fig. 3) showed PtO peaks at 2θ = 39.7° (111) in addition to the peaks for CeOz. The PtO peaks are shown in the inset of Fig. 3.

The PXRD diffractogram for the catalyst comprising ruthenium on ceria (Ru/CeOz, see Fig. 4) showed RuOz peaks at 2θ = 35.0° (101), 40.1° (200) and 54.3° (211) in addition to the peaks for CeOz. The RuOz peaks are shown in the insets of Fig. 4.

The PXRD diffractogram for the catalyst comprising palladium on ceria (Pd/CeOz, see Fig. 5) showed PdO peaks at 2θ = 42.6° (110) in addition to the peaks for CeOz. The PdO peaks are shown in the inset of Fig. 5.

Fig. 6 shows a reference spectrum for the ceria support.

XPS spectra of the respective catalysts shows the presence of the metal oxides $Rh_2O_3$ (see Fig. 7), IrOz (see Fig. 8) and PtO (see Fig. 9) on the catalysts M/CeOz. Fig. 8 also shows that some residual $IrCl_3$ starting material is left in the catalyst. Fig. 10 shows the XPS spectrum of RuOz for a Ru/CeOz catalyst. Also, in Fig. 10, residual $RuCl_3$ can be seen in addition to carbon impurities on the surface of the catalyst. Fig. 11 shows the XPS spectrum of PdO for a Pd/CeOz catalyst. As can be seen from Fig. 11, residual PdClz starting material is left in the catalyst. Figures 7 to 11 show the experimental curve in addition to the individual and the combined curves of a curve fit. Binding energy values of $Rh_2O_3$ (3d) was measured at 309.4 and 314 eV, of IrOz (4f) was measured at 61.9 and 64.8 eV, of PtO (4f) was measured at 73.5 and 76.7 eV, of RuOz (3d) was measured at 280.7, 281.1, 285 and 285.4 eV, and of PdO (3d) was measured at 336.8 and 342 eV.

Table 1 below shows the loading in wt.% of the metal as determined by ICP-MS analysis of the catalysts and the mean diameter of the catalyst nanoparticles as determined by electron microscopy.

Table 1: ICP-MS analysis of metal loading for M/CeOz.

| $M/CeO_2$ catalyst | wt. loading of M (%) | mean diameter (nm) |
|---|---|---|
| $Ru/CeO_2$ | 4.692 | 8.9 ± 0.2 |
| $Rh/CeO_2$ | 3.650 | 1.90 ± 0.02 |
| $Pd/CeO_2$ | 3.823 | 7.7 ± 0.2 |
| $Ir/CeO_2$ | 4.325 | 1.13 ± 0.01 |
| $Pt/CeO_2$ | 5.232 | 0.94 ± 0.01 |

Fig. 12a shows an electron micrograph of the catalyst Ru/CeOz. Fig. 12b shows an EDX analysis spectrum showing the presence of ruthenium on the ceria particle. Fig. 12c further shows a histogram of the nanoparticle size of the catalyst Ru/CeOz.

Fig. 13a shows an electron micrograph of the catalyst Rh/CeOz. Fig. 13b shows an EDX analysis spectrum showing the presence of rhodium on the ceria particle. Fig. 13c further shows a histogram of the nanoparticle size of the catalyst Rh/CeOz.

Fig. 14a shows an electron micrograph of the catalyst Ir/CeOz. Fig. 14b shows an EDX analysis spectrum showing the presence of iridium on the ceria particle. Fig. 14c further shows a histogram of the nanoparticle size of the

catalyst Ir/CeOz.

Fig. 15a shows an electron micrograph of the catalyst Pt/CeOz. Fig. 15b shows an EDX analysis spectrum showing the presence of platinum on the ceria particle. Fig. 15c further shows a histogram of the nanoparticle size of the catalyst Pt/CeOz.

Fig. 16a shows an electron micrograph of the catalyst Pd/CeOz. Fig. 16b shows an EDX analysis spectrum showing the presence of palladium on the ceria particle. Fig. 16c further shows a histogram of the nanoparticle size of the catalyst Pd/CeOz.

General procedure for the hydrogenolysis

In a typical hydrogenolysis reaction, the catalyst (0.5 wt% of metal to polymer) and 500 mg of the polymer to be subjected to hydrogenolysis were added to a glass vial (20 mL) with a glass-coated magnetic stir-bar. The vial was placed inside a Parr autoclave (68 mL), which was then purged three times with Hz and pressurized to the target Hz pressure (50 bar). The autoclave was placed in a Parr ceramic heater preheated to the target temperature (275-350 °C) under 700 r.p.m. stirring, for the chosen reaction time (1-24 h). After the reaction, the autoclave was placed under a running water-bath and cooled to room temperature.

Gaseous products except for ammonia were collected in a gas sampling bag and a fraction of 0.1 mL of this gas was analyzed by gas chromatography with a flame ionization detector (GC-FID). The total percentage of gas was then calculated based on this volume. At the end of the reaction, diethyl ether was used to extract liquid products while the solid residue was dried and weighted. Overall conversion was calculated from the mass difference at the end of the reaction as [restarting polymer) - m(solid products)]/[m(starting polymer)], hence solid products were not considered as conversion. Yield of each hydrocarbon product is calculated from the mole of carbon in the product divided by the mole of carbon in the starting polymer. Analysis of the extracted liquid products was performed on an Agilent 7000C GC-MS instrument using an Agilent DB-17 (50%-Phenyl)- methylpolysiloxane column with p-xylene as the internal standard.

For the GC-FID analysis, the alkane products were identified by comparison with the gas chromatograms of pure $C_1$-$C_6$ alkane or a mixture thereof.

In order to determine the amount of hexane in the reaction mixture, which hexane was isolated from the reaction mixture using diethyl ether, a calibration curve was recorded to measure the amount of n-hexane present in the liquid product, using 4 standard solutions with different p-xylene/n-hexane ratios. The calibration curve is shown in Fig. 17. The x-axis represents the FID integration area of p-xylene/ n-hexane while they-axis represents the real mass ratio of p-xylene/n-hexane.

Ammonia detection and quantification

To test for the presence of ammonia in the products, 5 mL of the gaseous product was extracted at ambient pressure with a gas syringe and slowly bubbled into 10 mL miliQ water. Ammonia standards were prepared by bubbling different volumes of pure ammonia gas into 10 mL miliQ water. The indophenol reagent was prepared following a previously reported method (Kempers, A. J.; Kok, C. J. Analytica Chimica Acta 1989, 221, 147-155). After adding the indophenol reagent, the samples were left to stand in the dark for 1h before measurement on a PerkinElmer Lambda 850+ UV-Vis spectrophotometer. The characteristic maximum absorbance peak of the indophenol blue complex at 651 nm was recorded and compared with the standards.

A calibration curve was prepared from four ammonia standards, with concentrations of 2.1, 4.1, 12.2 and 20.3 μM, prepared by gaseous injection and is shown in Fig. 18.

Then, using the ideal gas equation, PV = nRT, was used to calculate the total volume of gas present at the end of the reaction under 1 atm of pressure. As only a fraction (5 mL) of this volume was used, the real amount of ammonia was obtained as shown in the following equation:

$$Total\ n(NH_3) = ([NH_3] \times 10\ mL) \times \frac{overall\ V\ at\ 1\ atm}{5\ mL}$$

The skilled person is aware that in the method according to the invention, trace amounts of gaseous alkylamine byproducts may lead to increased ammonia values due to the sensitivity of the detection method.

Conversion of polyamide resins

The chemical structures of the polyamide resins converted are shown below

PA-6

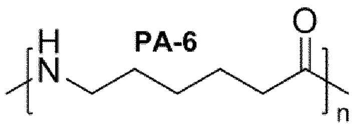

PA-66

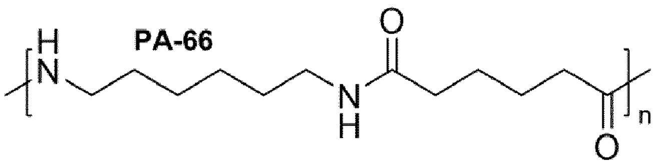

PA-12

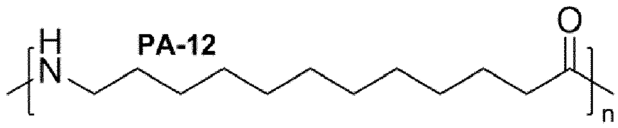

PA-612

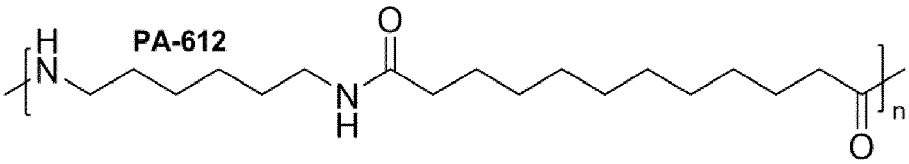

The results of the hydrogenolysis of the polyamide resins with Pt/CeOz are shown in Table 2 below. Ammonia was also produced in all catalysis examples described herein and was easily separated from the other compounds.

Table 2: Hydrogenolysis of various PA resins with $Pt/CeO_2$.

| entry | polymer | conv. (%) | $CH_4$ (%) | $C_2H_6$ (%) | $C_3H_8$ (%) | $C_4H_{10}$ (%) | $C_5H_{12}$ (%) | $C_6H_{14}$ (%) | others* (%) |
|---|---|---|---|---|---|---|---|---|---|
| 1 | PA-6 | 88 | 6 | <1 | <1 | 1 | 21 | 59 | 1 |
| 2 | PA-66 | 78 | 4 | <1 | <1 | 1 | 20 | 49 | 4 |
| 3 | PA-12 (i) | 98 | 5 | - | - | - | - | - | - |
| 4 | PA-612 (ii) | 99 | 6 | <1 | 1 | 1 | 8 | 29 | - |

Reaction conditions: polymer (500 mg), catalyst (2.5 mg Pt) and Hz (50 bar) at 350°C for 24h. *Includes ether soluble oligomers detected by GC-MS. Insoluble oligomers were not considered as a fraction of conversion.
(i) $C_{12}H_{26}$ = 60%, $C_{11}H_{24}$ = 33%
(ii) $C_{12}H_{26}$ = 30%, $C_{11}H_{24}$ = 18%, $C_{10}H_{22}$ - $C_7H_{16}$ = 6%

As can be seen from Table 2, the hydrogenolysis of the polyamide resins proceeds with high conversion, thereby producing alkanes that correspond to the number of carbon atoms of the repeat units in good yields.

Hydrogenolysis of the polyamide resins with the catalyst Ru/CeOz is shown in Table 3 below. As can be seen, the main product is methane, only small amounts of propane are formed, and butane and higher alkanes are not formed at all.

Table 3: Hydrogenolysis of various PA resins with $Ru/CeO_2$.

| entry | polymer | conv. (%) | $CH_4$ (%) | $C_2H_6$ (%) | $C_3H_8$ (%) | $C_4H_{10}$ (%) | $C_5H_{12}$ (%) | $C_6H_{14}$ (%) |
|---|---|---|---|---|---|---|---|---|
| 5 | PA-6 | 100 | 96 | 4 | - | - | - | - |
| 6 | PA-66 | 98 | 81 | 15 | 2 | - | - | - |
| 7 | PA-12 | 100 | >99 | <1 | - | - | - | - |

(continued)

| entry | polymer | conv. (%) | $CH_4$ (%) | $C_2H_6$ (%) | $C_3H_8$ (%) | $C_4H_{10}$ (%) | $C_5H_{12}$ (%) | $C_6H_{14}$ (%) |
|---|---|---|---|---|---|---|---|---|
| 8 | PA-612 | 100 | >99 | <1 | - | - | - | - |
| Reaction conditions: polymer (500 mg), catalyst (2.5 mg Ru) and Hz (50 bar) at 350°C for 24h. | | | | | | | | |

The effect of the duration of the hydrogenolysis is shown in Table 4 below for Pt/CeOz and Ru/CeOz.

Table 4: Conversion of PA-6 for different durations.

| entry | catalyst | time (h) | conv. (%) | $CH_4$ (%) | $C_2H_6$ (%) | $C_3H_8$ (%) | $C_4H_{10}$ (%) | $C_5H_{12}$ (%) | $C_6H_{14}$ (%) | others* (%) |
|---|---|---|---|---|---|---|---|---|---|---|
| 9 | $Ru/CeO_2$ | 2 | 100 | 86 | 8 | 4 | 2 | - | - | - |
| 10 | $Ru/CeO_2$ | 24 | 100 | 96 | 4 | - | - | - | - | - |
| 11 | $Pt/CeO_2$ | 12 | 65 | 2 | - | <1 | 1 | 11 | 34 | 17 |
| 12 | $Pt/CeO_2$ | 18 | 86 | 4 | - | <1 | 1 | 15 | 64 | 2 |
| 13 | $Pt/CeO_2$ | 24 | 88 | 6 | <1 | <1 | 1 | 21 | 59 | 1 |
| Reaction conditions: PA-6 (500 mg), catalyst (2.5 mg of metal) and Hz (50 bar) at 350°C. *Includes ether soluble oligomers detected by GC-MS. Insoluble oligomers were not considered as a fraction of conversion. | | | | | | | | | | |

Ammonia yield from three repeated reactions assuming the repeating unit represents the entire polymer.

Ru/CeOz = 66 ± 11 %
Pt/CeOz = 57 ± 16 %

Conversion of polyamide materials

The conversion of different polyamide materials using the method according to the invention was investigated. The results of the hydrogenolysis using Pt/CeOz are shown in Table 5 below.

Table 5: Detailed conversions from the hydrogenolysis of PA composites and blends with $Pt/CeO_2$.

| entry | substrate | conv. (%) | $CH_4$ (%) | $C_2H_6$ (%) | $C_3H_8$ (%) | $C_4H_{10}$ (%) | $C_5H_{12}$ (%) | $C_6H_{14}$ (%) | non-alkane** (%) |
|---|---|---|---|---|---|---|---|---|---|
| 14 | 30% glass-fibre composite PA-6* | 87 | 7 | 1 | 1 | 2 | 7 | 30 | 39 |
| 15 | 30% carbon-fibre composite PA-66* | 73 | 3 | <1 | <1 | <1 | 3 | 7 | 60 |
| 16 | Ultramid® C40 L (PA-6/66) | 73 | 6 | <1 | <1 | 1 | 18 | 46 | 2 |
| 17 | PE/PA-6 blend (1:1) | 42 | 3 | - | - | 1 | 7 | 27 | 4 |
| 18[i] | 30% carbon-fibre composite PA-66* | 88 | 8 | <1 | <1 | 3 | 13 | 35 | 26 |
| Reaction conditions: polymer (500 mg), catalyst (2.5 mg Pt) and Hz (50 bar) at 350°C for 24h, i, 72h. Conversion of composites were calculated with only polymer mass.<br>**Non-alkane includes ether soluble oligomers detected by GC-MS. Insoluble oligomers were not considered as a fraction of conversion. | | | | | | | | | |

As can be seen from Table 5, the polyamide materials containing fibers and other polymers such as polyethylene

(PE) were converted in the method according to the invention to long alkanes such as hexane with good yields.

The results of the hydrogenolysis using the catalyst Ru/CeOz are shown in Table 6 below.

Table 6: Detailed conversions from the hydrogenolysis of PA composites and blends with Ru/$CeO_2$.

| entry | polymer | conv. (%) | $CH_4$ (%) | $C_2H_6$ (%) | $C_3H_8$ (%) | $C_4H_{10}$ (%) | $C_5H_{12}$ (%) | $C_6H_{14}$ (%) |
|---|---|---|---|---|---|---|---|---|
| 19 | 30% glass-fibre composite PA-6* | 99 | 80 | 9 | 4 | 4 | 1 | 1 |
| 20 | 30% carbon-fibre composite PA-66* | 97 | 63 | 7 | 7 | 10 | 7 | 3 |
| 21 | Ultramid® C40 L (PA-6/66) | 100 | 89 | 11 | - | - | - | - |
| 22 | PE/PA-6 blend (1:1) | 100 | 96 | 4 | - | - | - | - |
| Reaction conditions: polymer (500 mg), catalyst (2.5 mg Ru) and Hz (50 bar) at 350°C for 24h. *Conversion of composites were calculated with only polymer mass. | | | | | | | | |

As can be seen from Table 6, using the catalyst Ru/CeOz, the hydrogenolysis of the polyamide materials yielded mainly methane, and ethane. For entries 2 and 19, butane, pentane and hexane were observed, albeit in low yields.

Conversion of a commercial polyamide product

A fishing line made from PA-6 (MOMOI super soft 0.286 mm) was converted according to the invention and using a Ru/CeOz catalyst. The results are shown in Table 7 below.

Table 7: Conversion of PA-6 fishing line (MOMOI super soft 0.286mm). Fishing line was cut into smaller pieces (5cm in length) before conversion.

| catalyst | conv. (%) | $CH_4$ (%) | $C_2H_6$ (%) | $C_3H_8$ (%) | $C_4H_{10}$ (%) | $C_5H_{12}$ (%) | $C_6H_{14}$ (%) | others (%) |
|---|---|---|---|---|---|---|---|---|
| Ru/CeOz | 97 | 72 | 11 | 7 | 5 | 2 | <1 | - |
| Pt/CeOz | 54 | 1 | - | <1 | <1 | 5 | 41 | 7 |
| Reaction conditions: PA-6 fishing line (500 mg), catalyst (2.5 mg of metal) and Hz (50 bar) at 350°C for 40h. *Conversion may only account for ether soluble oligomers. | | | | | | | | |

As can be seen from Table 7, the Ru/CeOz catalyst mainly yields methane, Pt/CeOz also yields longer alkanes such as hexane in good yields.

**Claims**

**1.** A method for converting a polymer into hydrocarbon compounds, comprising the steps of

a. contacting the polymer with a catalyst to provide a reaction mixture, wherein the polymer contains nitrogen in the polymer backbone, wherein the catalyst comprises a transition metal on a carrier,
b. heating the reaction mixture in the presence of a reducing agent to a temperature of 50 to 1000°C,
c. recovering the hydrocarbon compounds from the reaction mixture.

**2.** The method of claim 1, **characterized in that** the polymer is selected from the group consisting of polyamides, polyurethanes, polyureas, polyamines, aminoplast resins, and copolymers and mixtures thereof, preferably selected from polyamides, polyurethanes, and copolymers and mixtures thereof, more preferably selected from polyamides.

**3.** The method according to any one of claims 1 or 2, **characterized in that** the transition metal is selected from iron group metals, cobalt group metals, nickel group metals, and mixtures thereof, preferably selected from the group

consisting of iron, cobalt, nickel, ruthenium, rhodium, platinum, iridium, and mixtures thereof, more preferably **in that** the transition metal is platinum or iridium, most preferably **in that** the transition metal is platinum, and/or wherein the transition metal is present in the form of a metal cluster or a metal oxide.

**4.** The method according to any one of the previous claims, **characterized in that** the carrier is zirconia, ceria, alumina, silica, zeolites such as zeolite-Y or ZSM-5, or carbon, preferably ceria or carbon, more preferably ceria.

**5.** The method according to any one of the previous claims, **characterized in that** the reducing agent is selected from the group consisting of hydrogen, sodium hydride, hydrazine, formic acid, hydrogen donors such as alcohols, for example isopropanol or glycerol, and saturated alkanes such as polyethylene and mixtures thereof, preferably **in that** the reducing agent is hydrogen, in particular hydrogen at a pressure from 1 to 200 bars, preferably from 25 to 150 bars, more preferably from 40 to 100 bars, most preferably from 40 to 60 bars.

**6.** The method according to any one of the previous claims, **characterized in that** the reaction mixture is heated in step b. to a temperature of 150 to 500°C, preferably from 200 to 500°C, more preferably from 250 to 400°C, even more preferably from 300 to 400°C, most preferably from 300 to 375 °C.

**7.** The method according to any one of the previous claims, **characterized in that** step a. is conducted without a solvent.

**8.** The method according to any one of claims 1 to 6, **characterized in that** step a. is conducted in a solvent, preferably an organic solvent, in particular C5-C16 saturated aliphatic compounds, more particularly C8-C12 saturated aliphatic compounds.

**9.** The method according to any one of the previous claims, **characterized in that** the hydrocarbon compounds have 1 to 20 carbon atoms, preferably 2 to 20 carbon atoms, and/or wherein the hydrocarbon compounds are selected from the group consisting of alkanes, cycloalkanes, alkenes, aromatic hydrocarbon compounds, and mixtures thereof, preferably selected from the group consisting of alkanes, cycloalkanes, aromatic hydrocarbon compounds, and mixtures thereof, more preferably selected from the group consisting of alkanes, cycloalkanes and mixtures thereof.

**10.** The method according to claim 9, **characterized in that** the alkanes have 1 to 20 carbon atoms, preferably 2 to 20 carbon atoms, more preferably 4 to 20 carbon atoms, and/or wherein the cycloalkanes have 3 to 20 carbon atoms, preferably 3 to 10 carbon atoms, and/or wherein the alkenes have 2 to 20 carbon atoms, preferably 4 to 20 carbon atoms, and/or wherein the aromatic hydrocarbon compounds have 5 to 20 carbon atoms, preferably 5 to 15 carbon atoms.

**11.** The method according to any one of the previous claims, **characterized in that** the hydrocarbon compounds comprise methane in an amount of 60 wt.% or less, preferably 40 wt.% or less, more preferably 20 wt.% or less, most preferably 5 wt.% or less, based on the total weight of the hydrocarbon compounds.

**12.** The method according to any one of the previous claims, **characterized in that** the polymer comprises at least one type of repeat units and the hydrocarbon compounds comprise an alkane, a cycloalkane, or an aromatic compound having the same number of carbon atoms as the at least one type of repeat units of the polymer, in an amount of at least 10 wt.%, preferably in an amount of at least 20 wt.%, more preferably in an amount of at least 30 wt.%, even more preferably in an amount of at least 40 wt.%, most preferably in an amount of at least 50 wt.%, based on the total weight of the hydrocarbon compound.

**13.** The method according to any one of the previous claims, **characterized in that** step b. is conducted for 2 to 80 hours, preferably 5 to 60 hours, more preferably 8 to 48 hours, most preferably 10 to 36 hours.

**14.** The method according to any one of the previous claims, **characterized in that** the transition metal is present in an amount of 0.001 to 5 wt.%, preferably 0.01 to 5 wt.%, more preferably 0.05 to 3 wt.%, most preferably 0.1 to 2 wt.%, based on the amount of the polymer.

**15.** The method according to any one of the previous claims, **characterized in that** ammonia and/or hydrocarbon amines, in particular ammonia, is recovered as additional product.

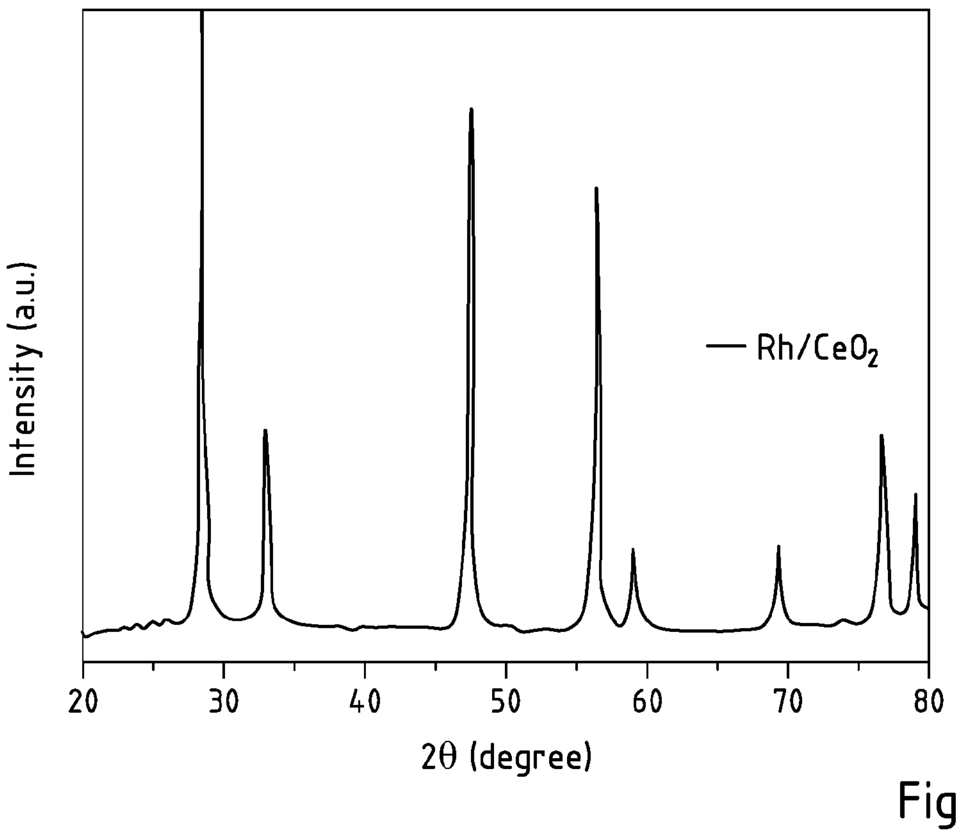

Intensity (a.u.)
Rh/CeO2
20 30 40 50 60 70 80
2θ (degree)


Fig.1

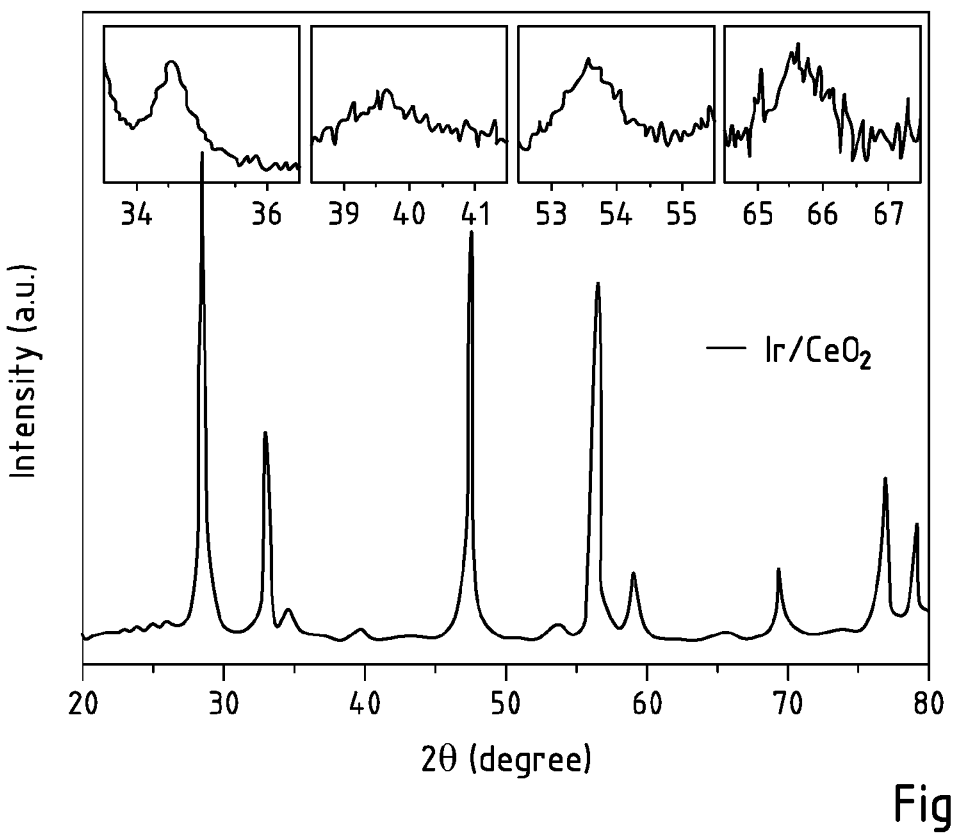

34 36
39 40 41
53 54 55
65 66 67
Intensity (a.u.)
Ir/CeO2
20 30 40 50 60 70 80
2θ (degree)


Fig.2

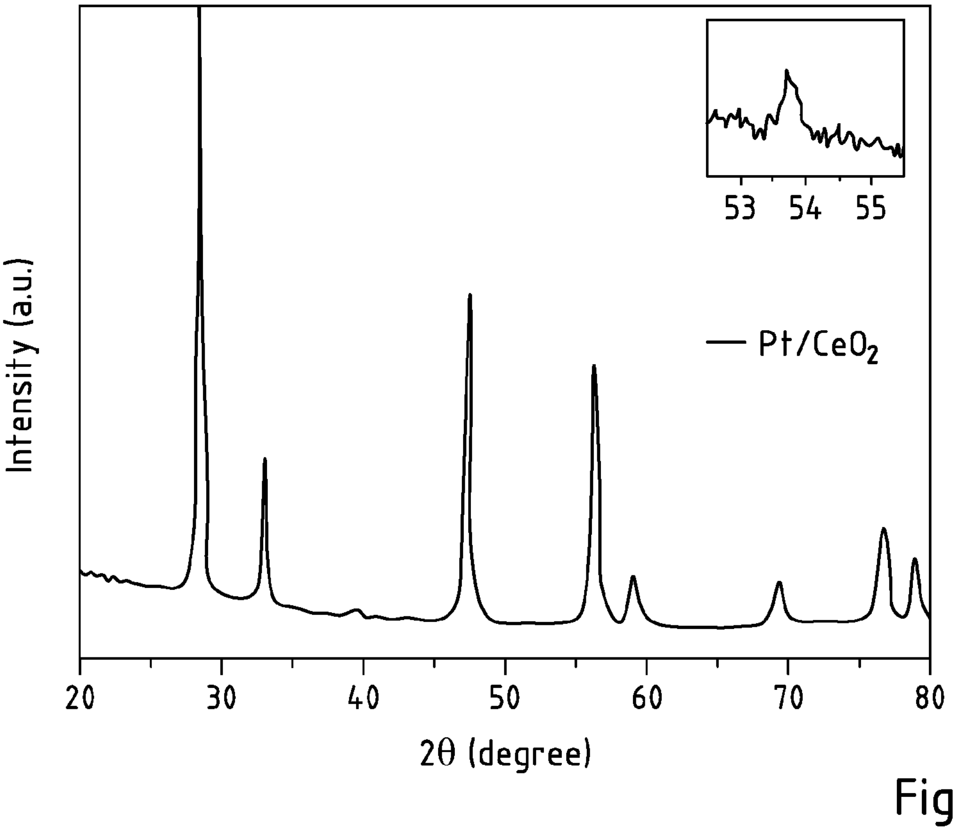

Intensity (a.u.)
Pt/CeO2
53
54
55
20
30
40
50
60
70
80
2θ (degree)

Fig.3

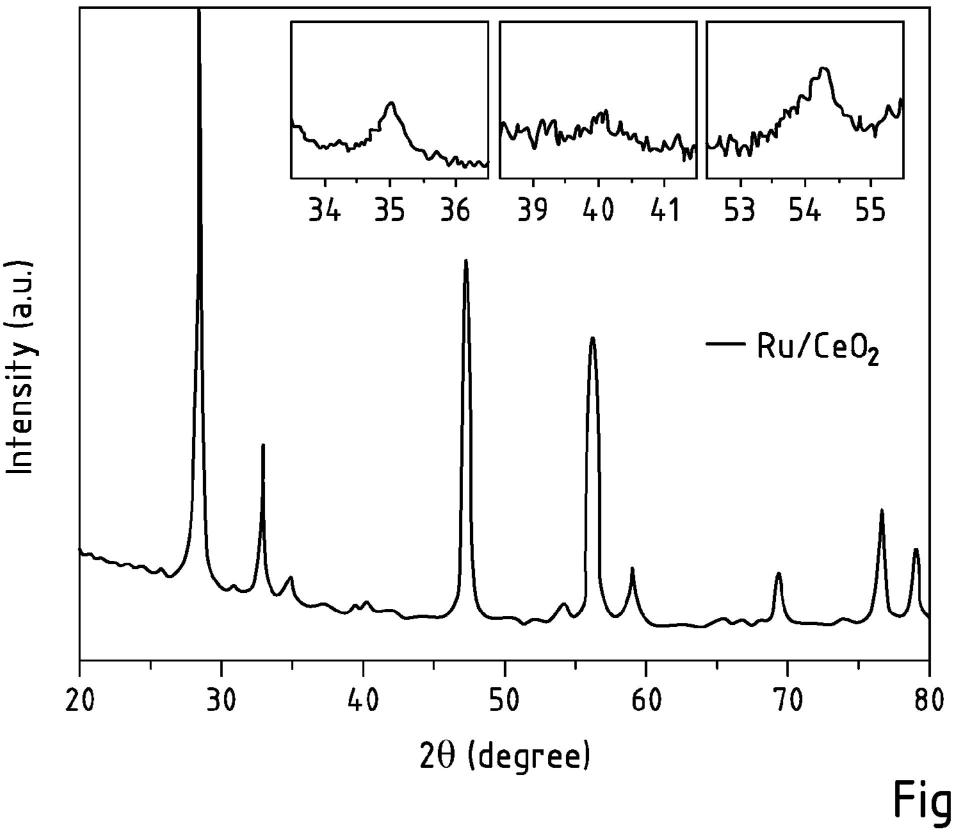

Intensity (a.u.)
Ru/CeO2
34
35
36
39
40
41
53
54
55
20
30
40
50
60
70
80
2θ (degree)

Fig.4

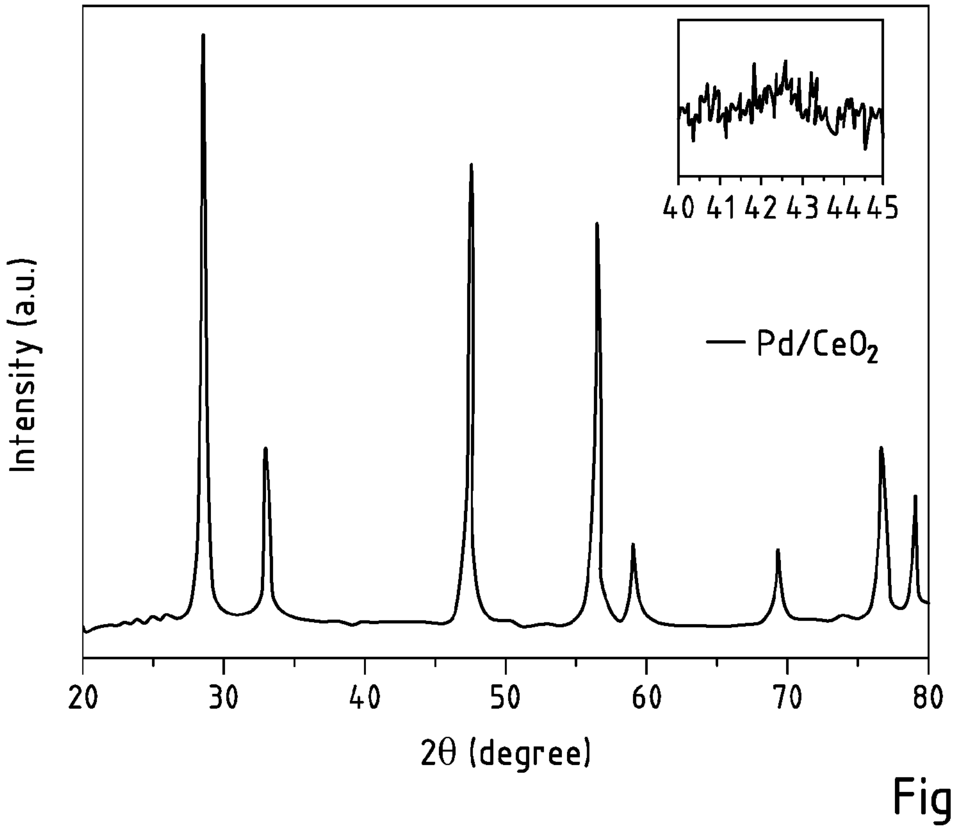
Intensity (a.u.)
2θ (degree)
20
30
40
50
60
70
80
Pd/CeO2
40 41 42 43 44 45

Fig.5

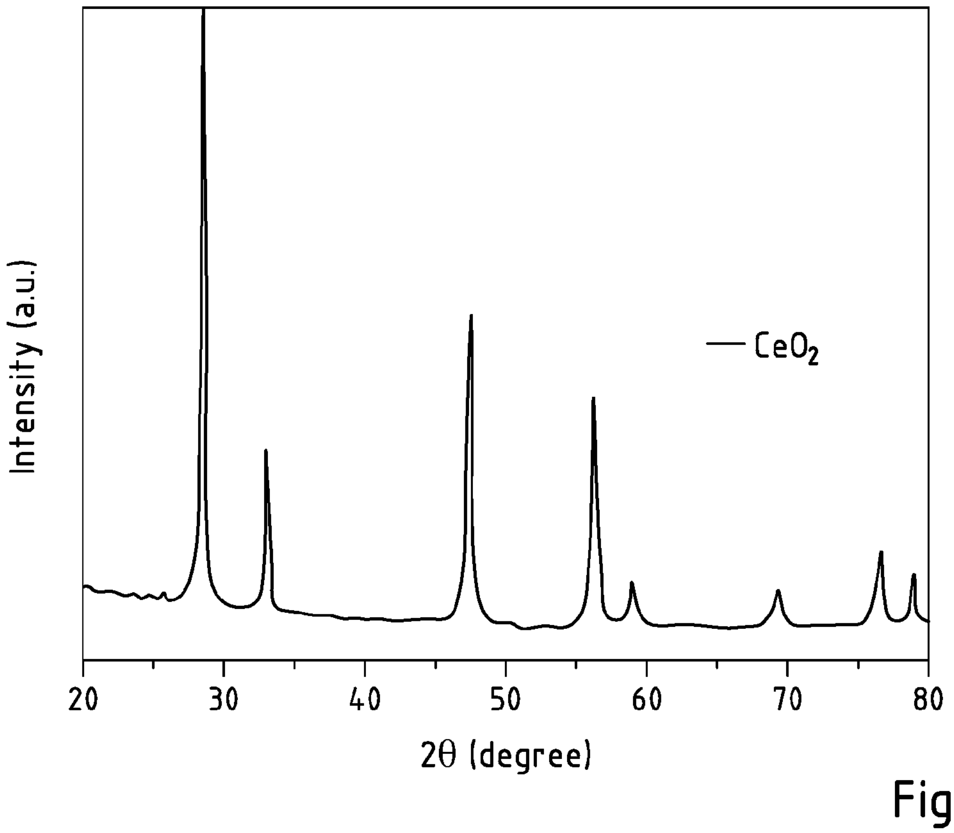
Intensity (a.u.)
2θ (degree)
20
30
40
50
60
70
80
CeO2

Fig.6

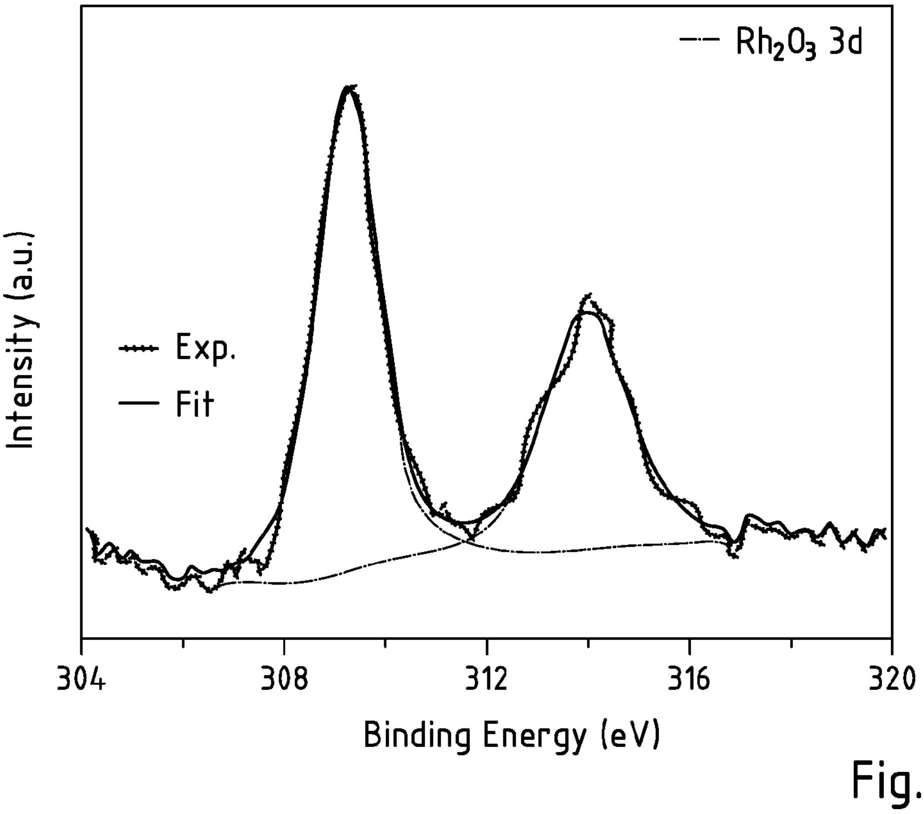
Rh$_2$O$_3$ 3d
Exp.
Fit
Intensity (a.u.)
304
308
312
316
320
Binding Energy (eV)

Fig.7

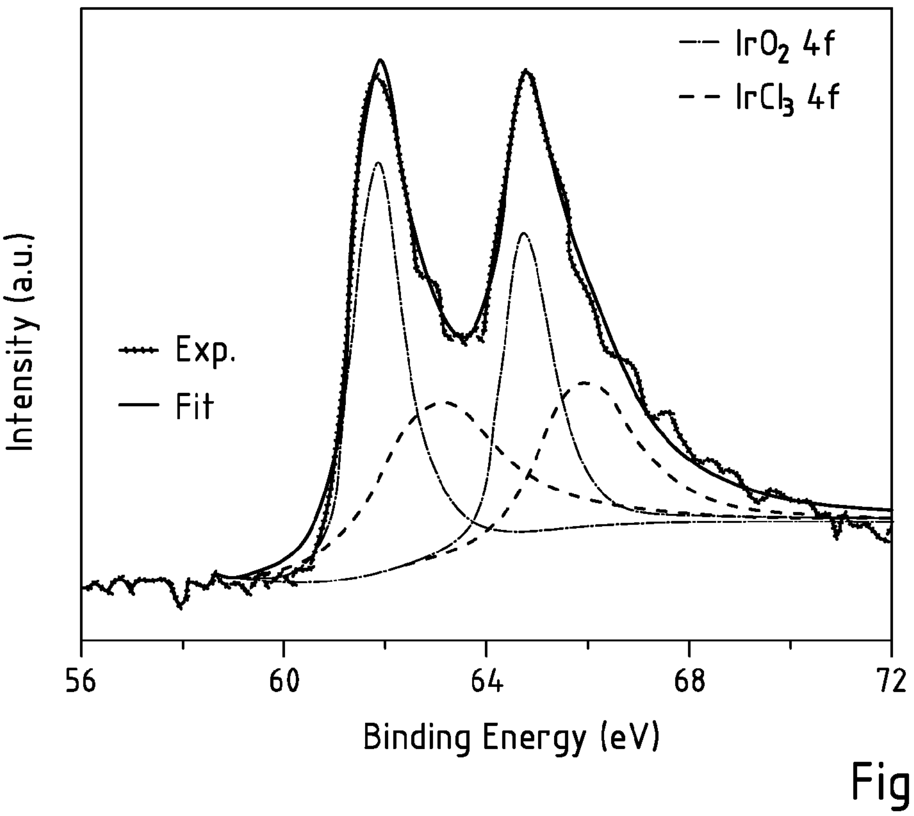
IrO$_2$ 4f
IrCl$_3$ 4f
Exp.
Fit
Intensity (a.u.)
56
60
64
68
72
Binding Energy (eV)

Fig.8

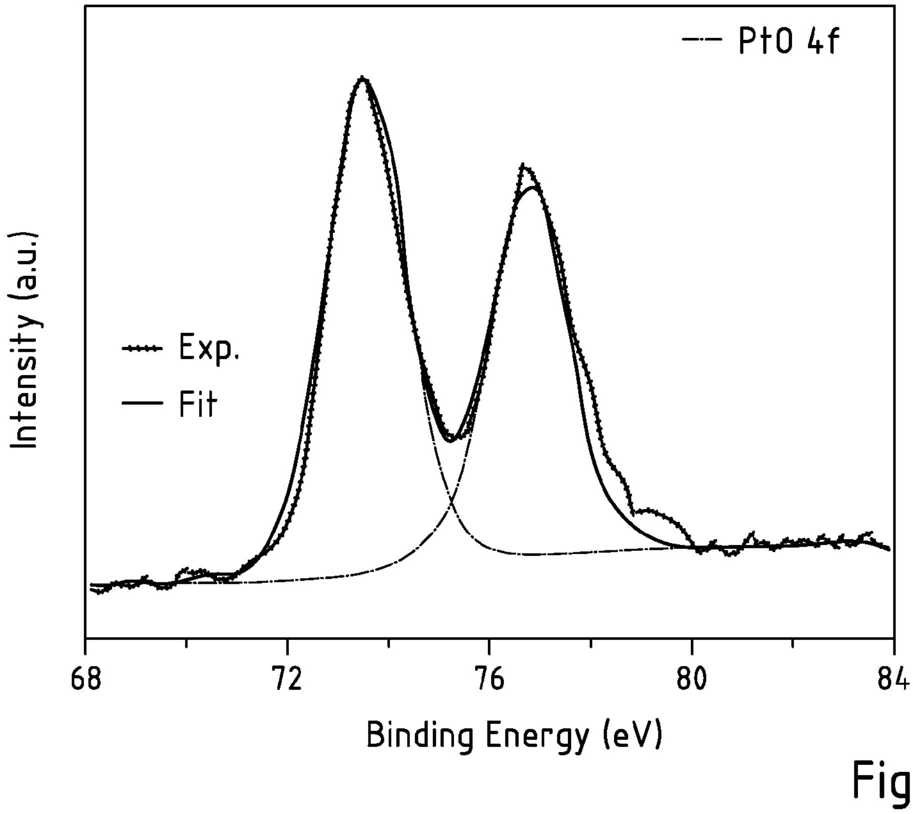
PtO 4f
Exp.
Fit
Intensity (a.u.)
68
72
76
80
84
Binding Energy (eV)

Fig.9

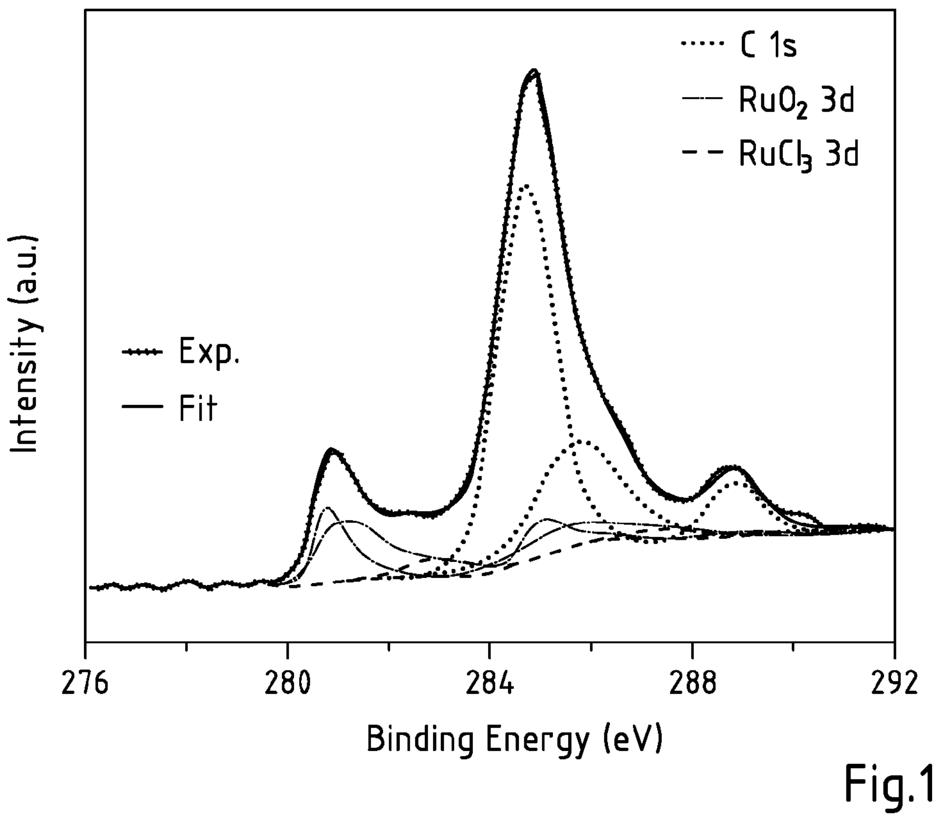
C 1s
RuO2 3d
RuCl3 3d
Exp.
Fit
Intensity (a.u.)
276
280
284
288
292
Binding Energy (eV)

Fig.10

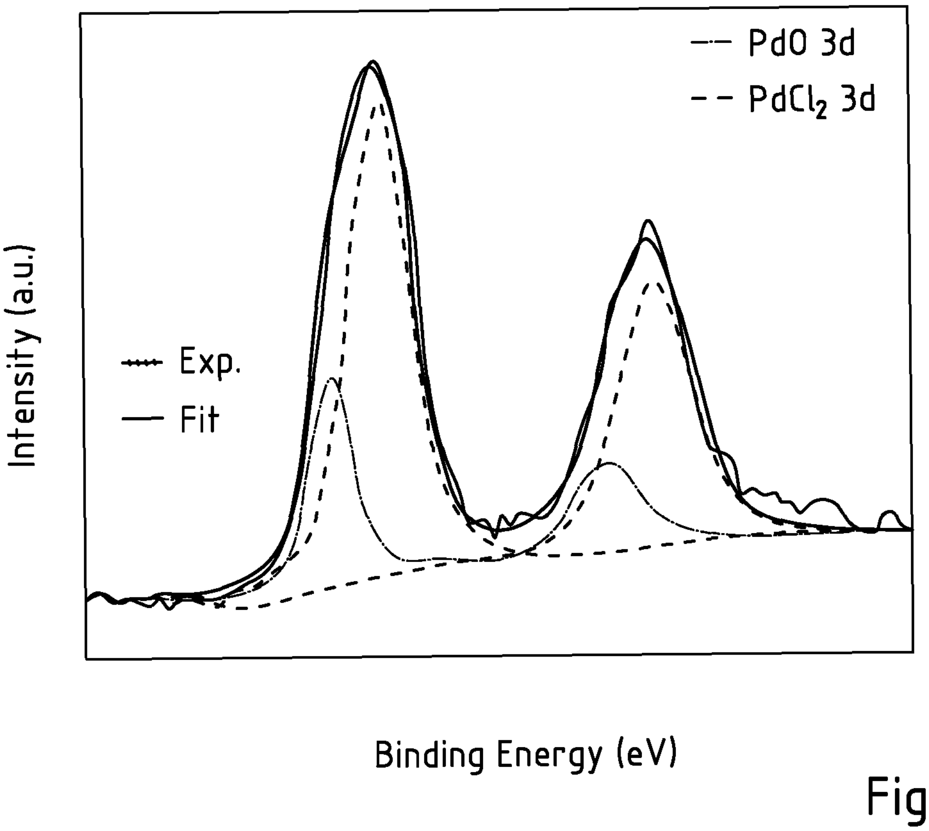


Fig.11

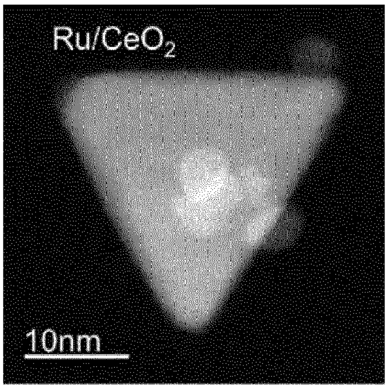


Fig.12a

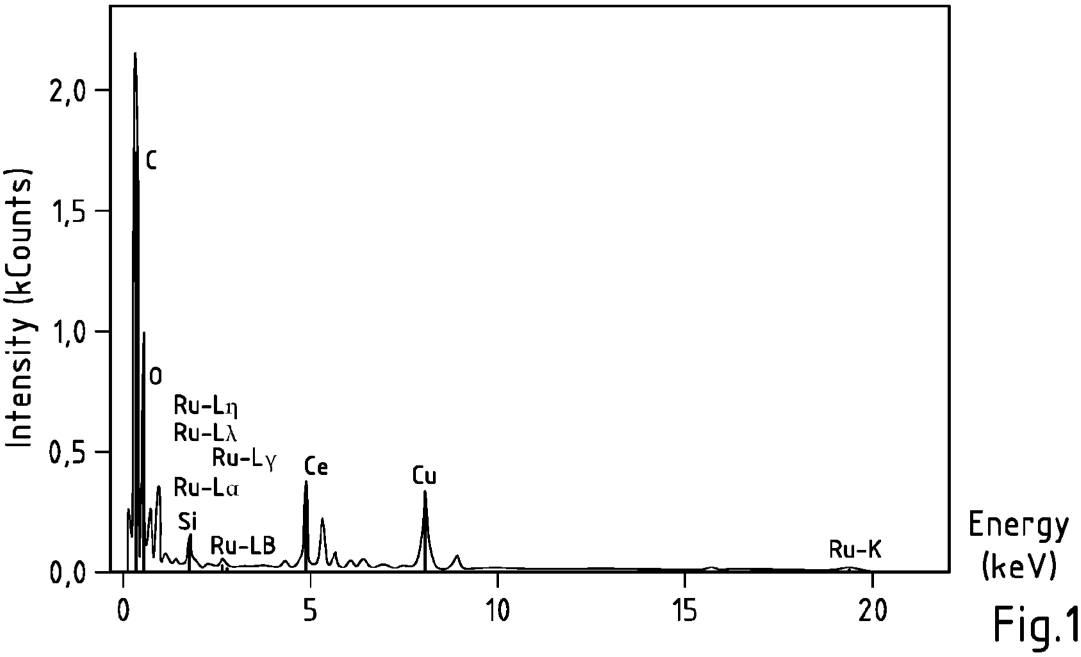


Fig.12b

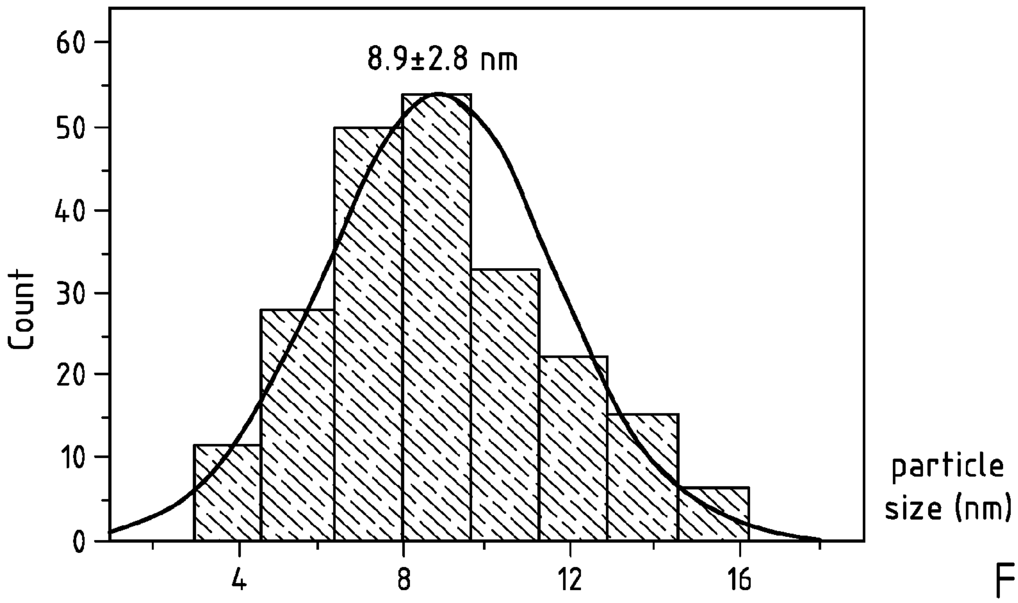


Fig.12c

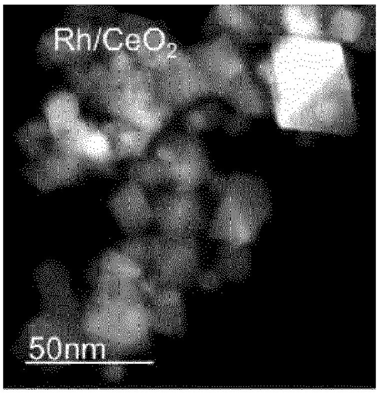


Fig.13a

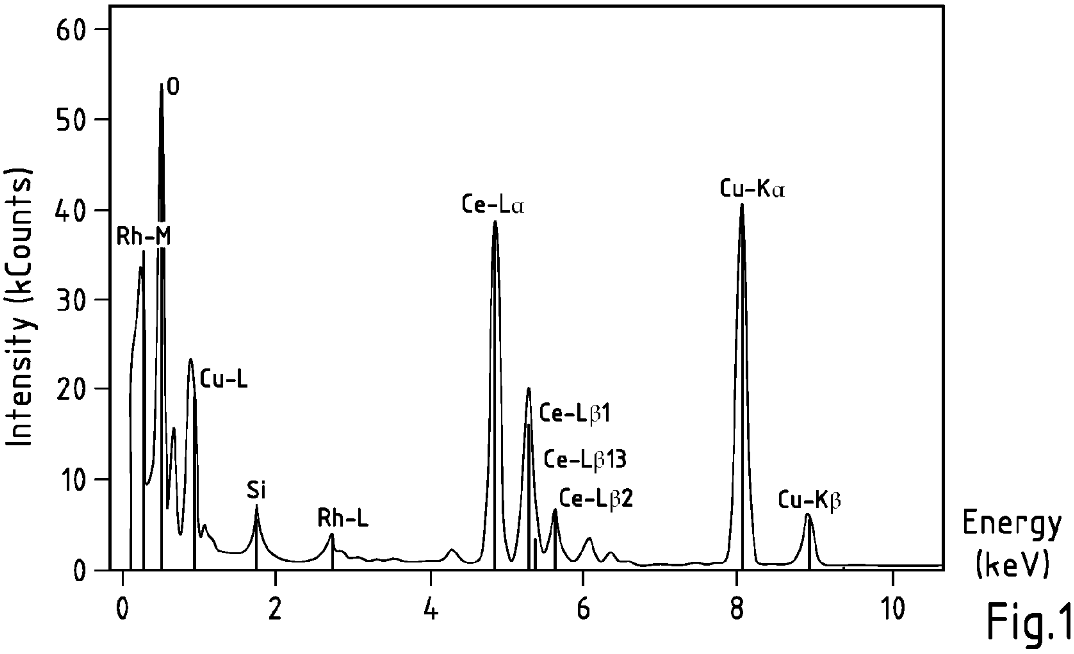


Fig.13b

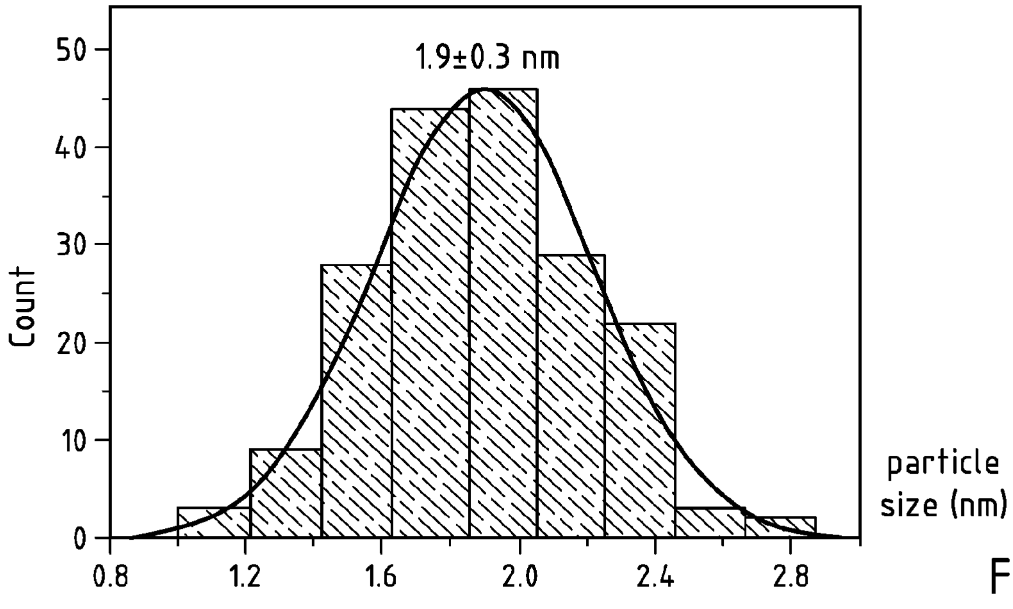


Fig.13c

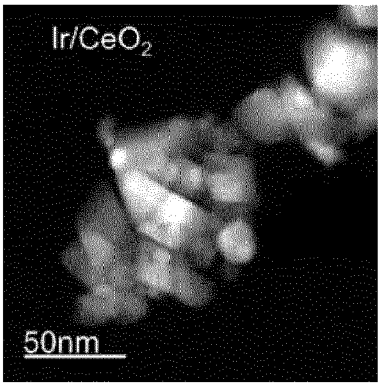


Fig.14a

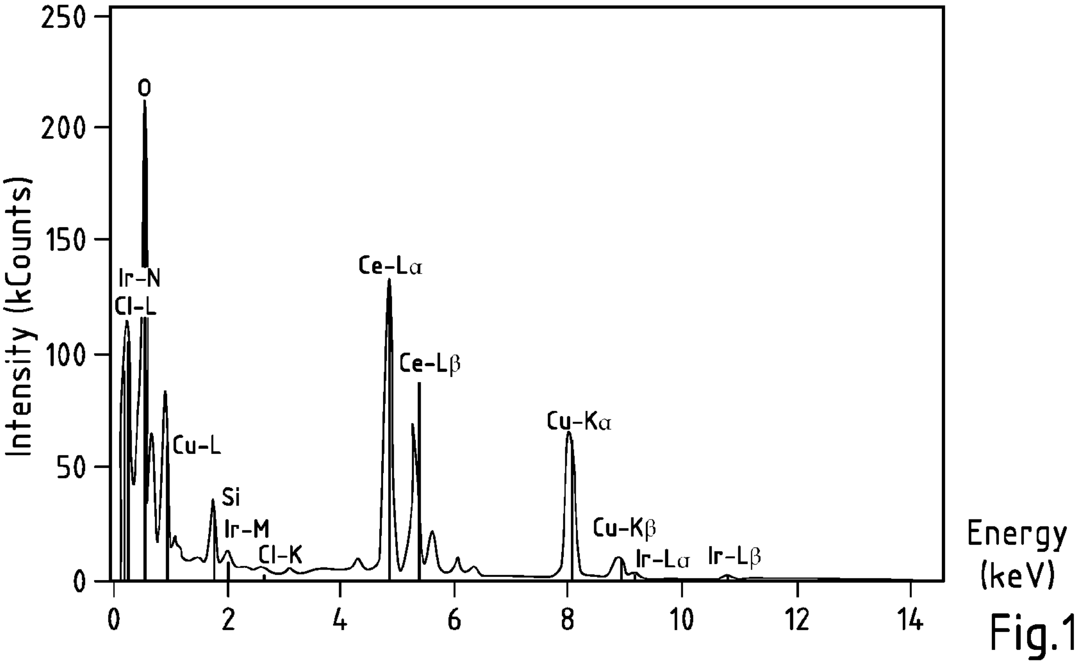


Fig.14b

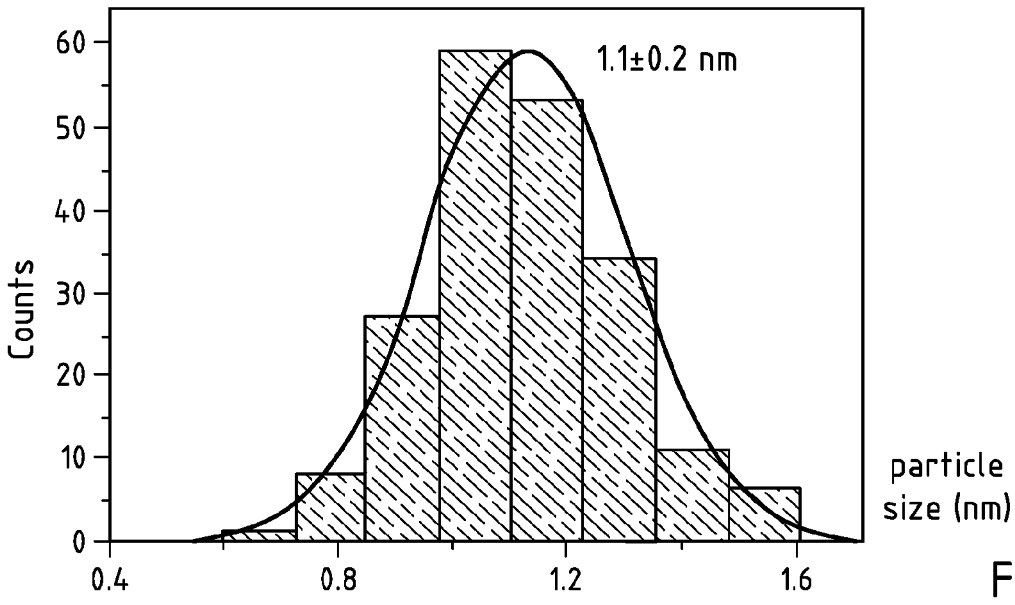


Fig.14c

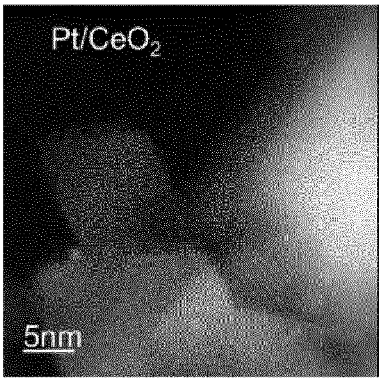


Fig.15a

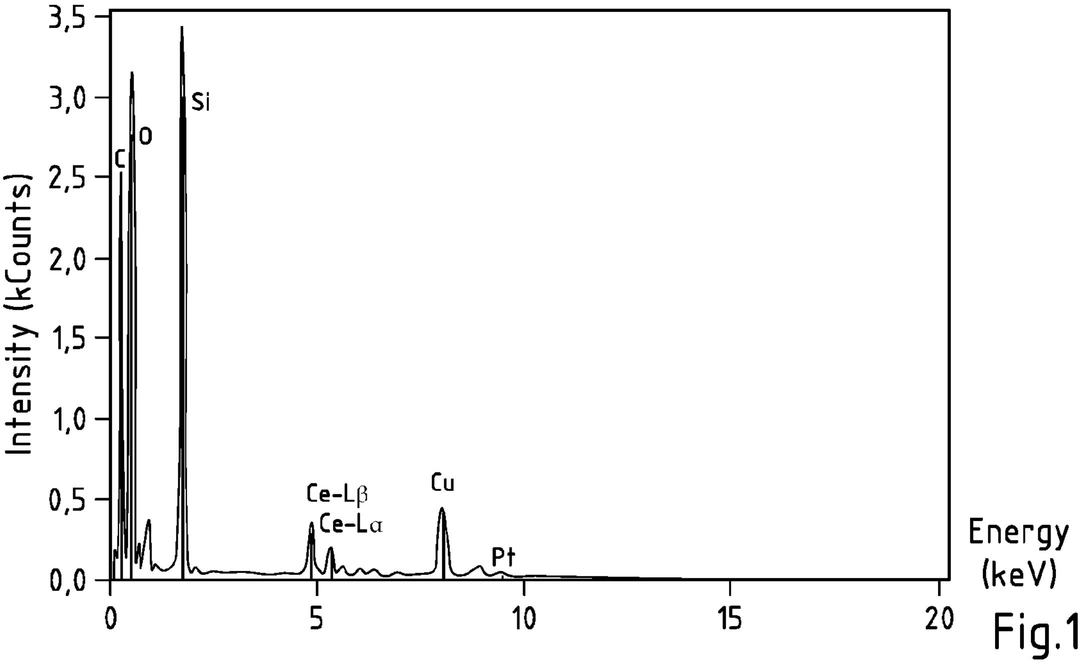


Fig.15b

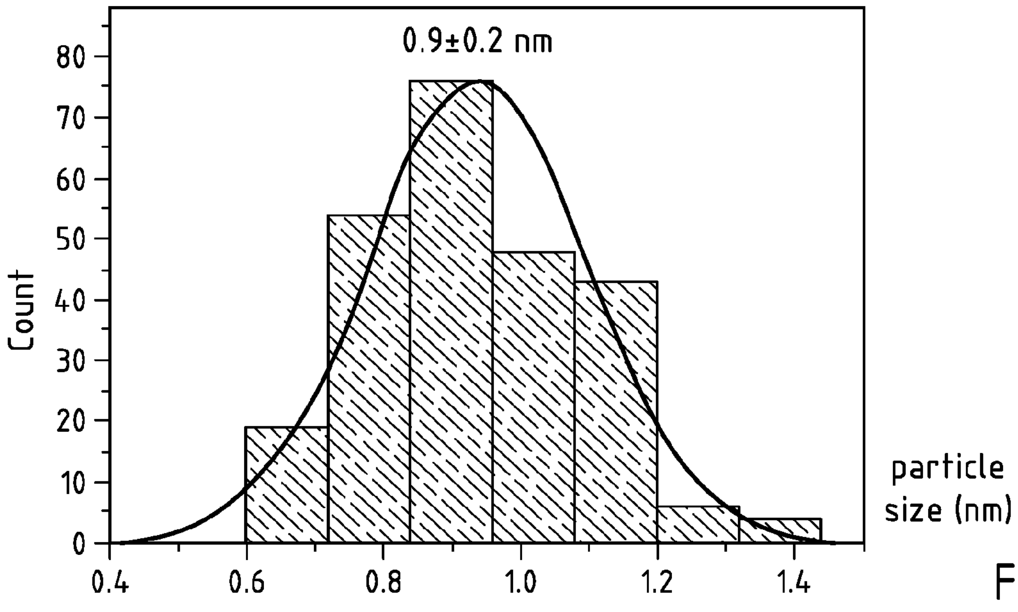


Fig.15c

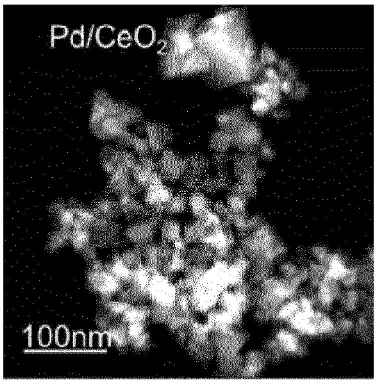

Fig.16a

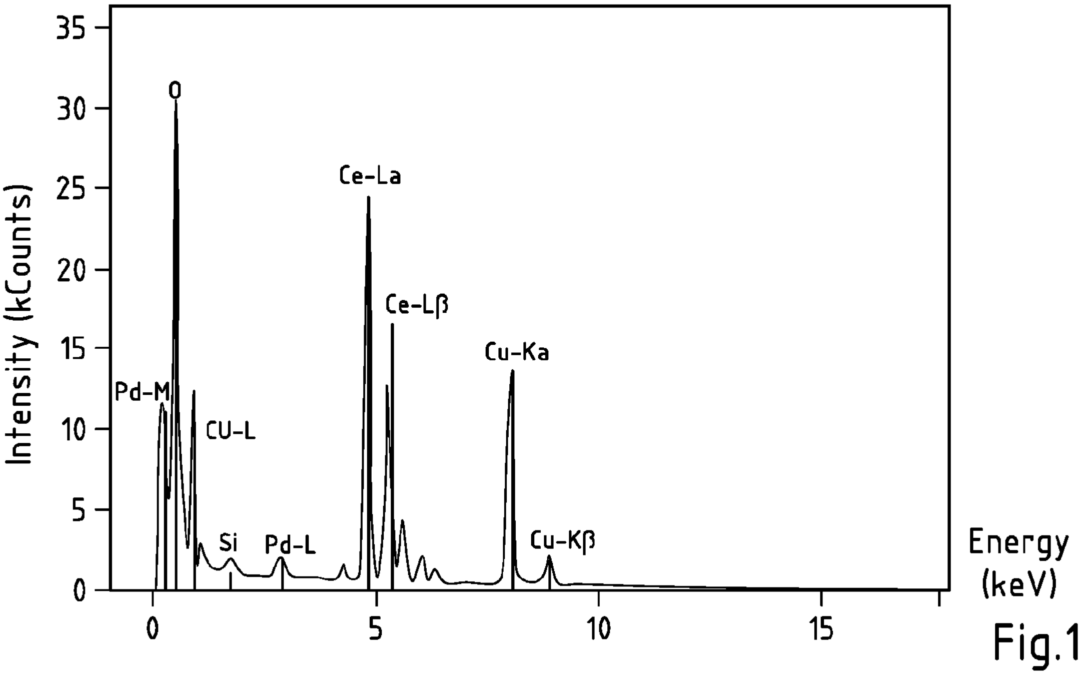

Fig.16b

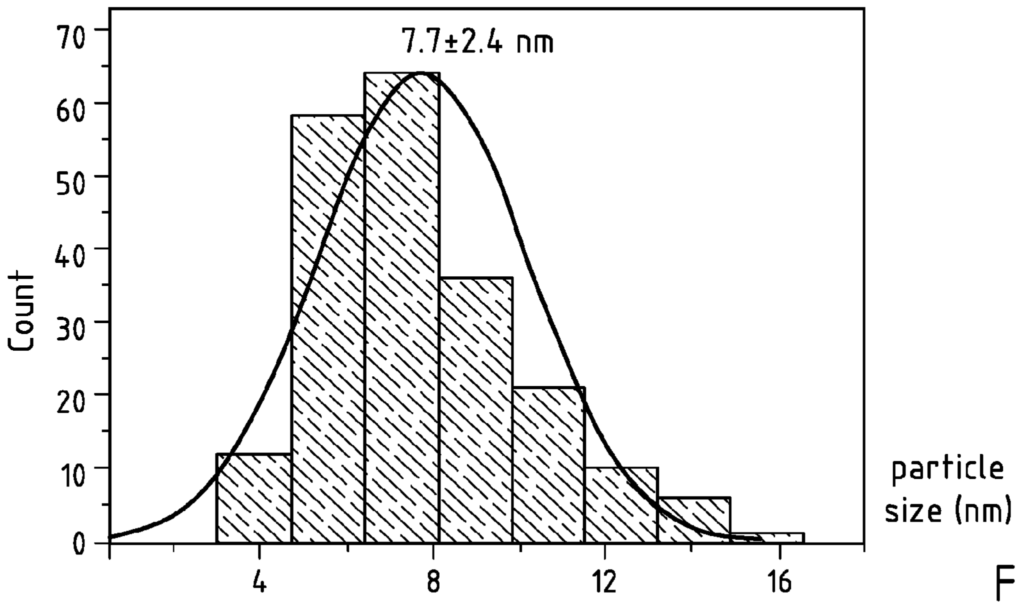

Fig.16c

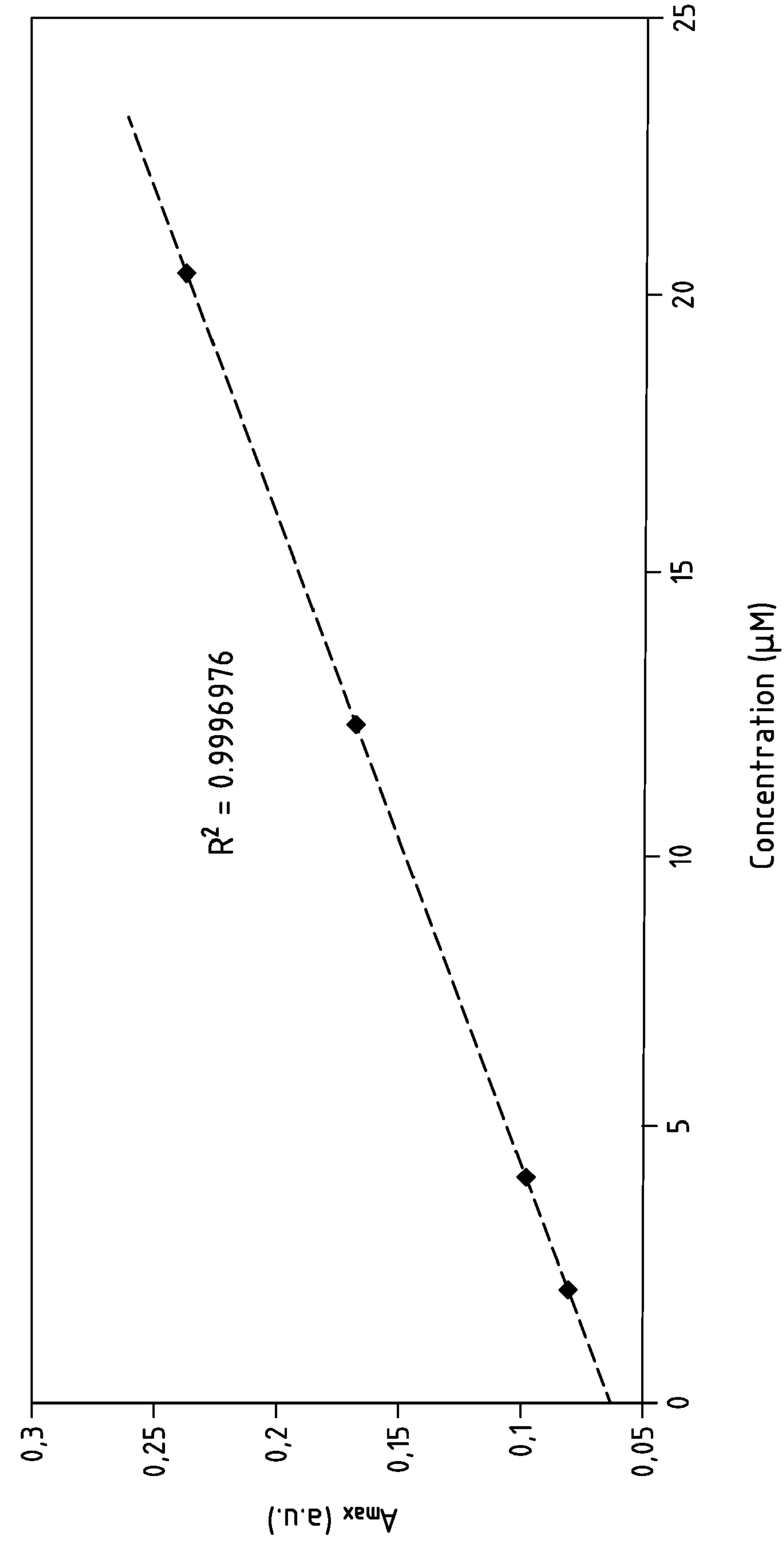

$R^2$ = 0.9996976
Concentration (µM)
0
5
10
15
20
25
$A_{max}$ (a.u.)
0,05
0,1
0,15
0,2
0,25
0,3


Fig.17

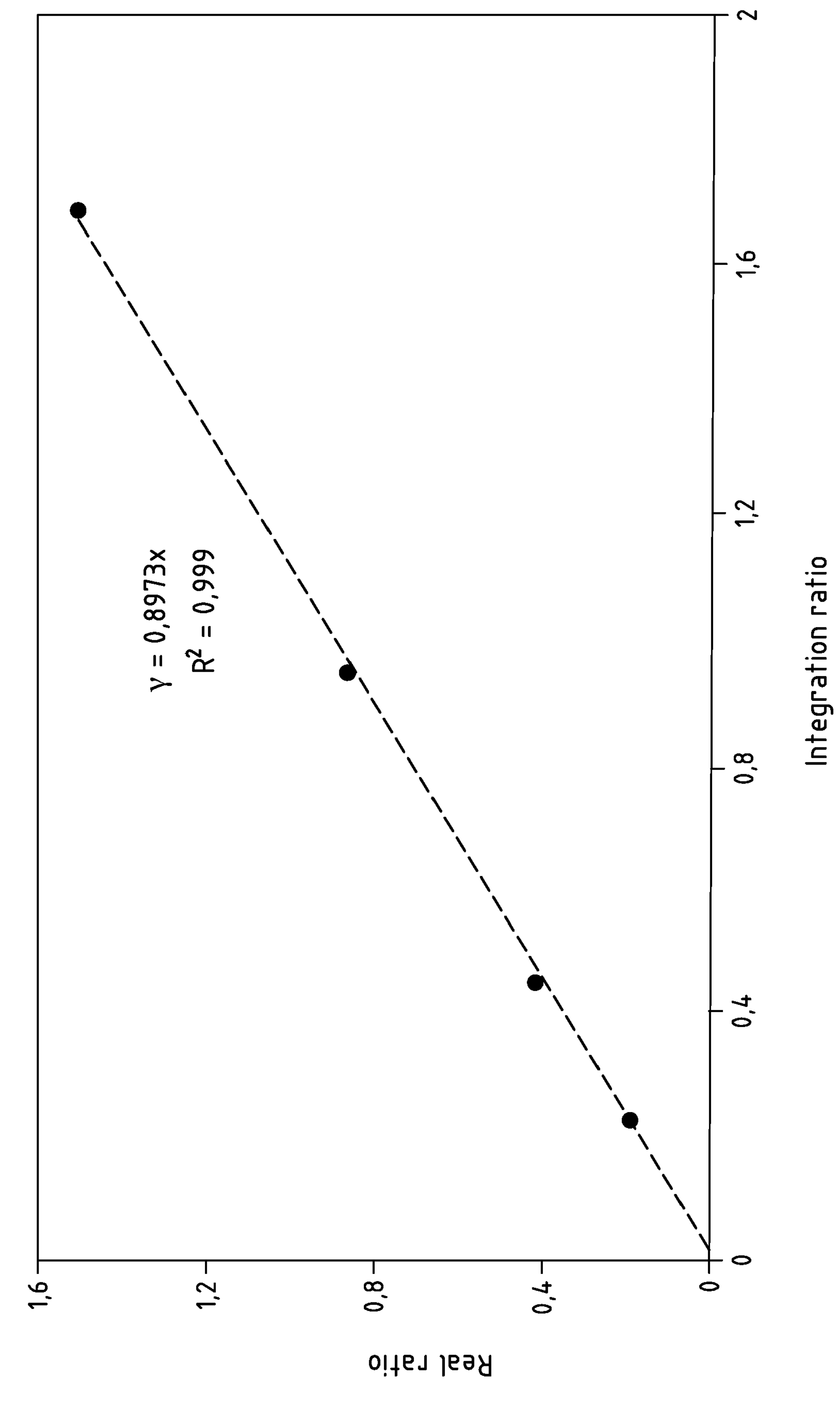

y = 0,8973x
R² = 0,999
Real ratio
0
0,4
0,8
1,2
1,6
Integration ratio
0
0,4
0,8
1,2
1,6
2


Fig.18

European Patent Office

# EUROPEAN SEARCH REPORT

Application Number
EP 23 15 9632

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CHEN LINXIAO ET AL: "Effect of reaction conditions on the hydrogenolysis of polypropylene and polyethylene into gas and liquid alkanes", REACTION CHEMISTRY & ENGINEERING, vol. 7, no. 4, 1 January 2022 (2022-01-01), pages 844-854, XP093075856, DOI: 10.1039/D1RE00431J Retrieved from the Internet: URL:http://pubs.rsc.org/en/content/articlepdf/2022/RE/D1RE00431J> * the whole document * | 1-15 | INV. C07C1/32 C07C9/04 C07C9/06 C07C9/14 C07C9/08 C07C9/10 C10G1/00 |
| A,D | CELIK GOKHAN ET AL: "Upcycling Single-Use Polyethylene into High-Quality Liquid Products", ACS CENTRAL SCIENCE, vol. 5, no. 11, 27 November 2019 (2019-11-27), pages 1795-1803, XP093075859, ISSN: 2374-7943, DOI: 10.1021/acscentsci.9b00722 * the whole document * | 1-15 | TECHNICAL FIELDS SEARCHED (IPC) C07C C10G |
| A | EP 3 653 687 A1 (ECOLE POLYTECHNIQUE FED DE LAUSANNE EPFL EPFL TTO [CH]) 20 May 2020 (2020-05-20) * claims 1-2 * * claim 4 * | 1-15 | |

The present search report has been drawn up for all claims

| Place of search | Date of completion of the search | Examiner |
|---|---|---|
| The Hague | 31 August 2023 | Seitner, Irmgard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

## ANNEX TO THE EUROPEAN SEARCH REPORT ON EUROPEAN PATENT APPLICATION NO.

EP 23 15 9632

This annex lists the patent family members relating to the patent documents cited in the above-mentioned European search report. The members are as contained in the European Patent Office EDP file on
The European Patent Office is in no way liable for these particulars which are merely given for the purpose of information.

31-08-2023

| Patent document cited in search report | Publication date | Patent family member(s) | Publication date |
|---|---|---|---|
| EP 3653687 A1 | 20-05-2020 | EP 3653687 A1 | 20-05-2020 |
| | | EP 3884013 A1 | 29-09-2021 |
| | | WO 2020104385 A1 | 28-05-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **CELIK, G. ; KENNEDY, R. M. ; HACKLER, R. A. ; FERRANDON, M. ; TENNAKOON, A. ; PATNAIK, S. ; LAPOINTE, A. M. ; AMMAL, S. C. ; HEYDEN, A. ; PERRAS, F. A.** *ACS Cent. Sci.,* 2019, vol. 5 (11), 1795-1803
- **CHEN, L. ; ZHU, Y. ; MEYER, L. C. ; HALE, L. V. ; LE, T. T. ; KARKAMKAR, A. ; LERCHER, J. A. ; GUTIÉRREZ, O. Y. ; SZANYI, J.** *React. Chem. Eng.,* 2022, vol. 7 (4), 844-854
- **KOTS, P. A. ; LIU, S. ; VANCE, B. C. ; WANG, C. ; SHEEHAN, J. D. ; VLACHOS, D. G.** *ACS Catal,* 2021, vol. 11 (13), 8104-8115
- **F. ZHANG et al.** *Science,* 2020, vol. 370 (6515), 437-441
- **HAI, X. ; XI, S. ; MITCHELL, S. ; HARRATH, K. ; XU, H. ; AKL, D. F. ; KONG, D. ; LI, J. ; LI, Z. ; SUN, T.** *Nature Nanotechnology,* 2021, https://doi.org/10.1038/s41565-021-01022-y
- **KEMPERS, A. J. ; KOK, C. J.** *Analytica Chimica Acta,* 1989, vol. 221, 147-155